(19) **Europäisches Patentamt / European Patent Office / Office européen des brevets**

(11) **EP 0 551 408 B2**

(12) # NEW EUROPEAN PATENT SPECIFICATION
After opposition procedure

(45) Date of publication and mention
of the opposition decision:
**15.08.2012 Bulletin 2012/33**

(45) Mention of the grant of the patent:
**09.02.2000 Bulletin 2000/06**

(21) Application number: **91918950.6**

(22) Date of filing: **04.10.1991**

(51) Int Cl.:
***C11D 3/386*** (2006.01)    ***D06M 16/00*** (2006.01)
***C11D 7/42*** (2006.01)

(86) International application number:
**PCT/US1991/007277**

(87) International publication number:
**WO 1992/006165 (16.04.1992 Gazette 1992/09)**

(54) **DETERGENT COMPOSITIONS CONTAINING CELLULASE COMPOSITIONS DEFICIENT IN CBH I TYPE COMPONENTS**

CELLULASEZUSAMMENSETZUNGEN MIT EINEM DEFIZIT AN KOMPONENTEN DES TYPS-CBH I ENTHALTENDE REINIGUNGSMITTELZUSAMMENSETZUNGEN

COMPOSITIONS DE DETERGENT CONTENANT DES COMPOSITIONS DE CELLULASE MANQUANT DE CONSTITUANTS DE TYPE CBH I

(84) Designated Contracting States:
**AT BE CH DE DK ES FR GB GR IT LI LU NL SE**

(30) Priority: **11.06.1991 US 713738**

(43) Date of publication of application:
**21.07.1993 Bulletin 1993/29**

(60) Divisional application:
**98111137.0 / 0 877 077**

(73) Proprietor: **GENENCOR INTERNATIONAL, INC.**
**South San Francisco, CA 94080 (US)**

(72) Inventors:
• **CLARKSON, Kathleen, A.**
**San Francisco, CA 94110 (US)**
• **WEISS, Geoffrey, L.**
**San Francisco, CA 94117 (US)**
• **LARENAS, Edmund, A.**
**Moss Beach, CA 94038 (US)**

• **SHOEMAKER, Sharon, P.**
**Fairfield, CA 94533 (US)**

(74) Representative: **Armitage, Ian Michael et al**
**Mewburn Ellis LLP**
**33 Gutter Lane**
**London**
**EC2V 8AS (GB)**

(56) References cited:
WO-A1-89/09259    WO-A1-91/05841
US-A- 4 435 307    US-A- 4 978 470

• **WOOD ET AL.: 'Methods of Measuring Cellulase Activities' METHODS IN ENZYMOLOGY vol. 160, 1988, pages 87 - 112, XP002088718**
• **COUGHLAN; ET AL: 'Comparative Biochemistry of Fungal and Bacterial Cellulolytic Enzyme Systems' BIOCHEMISTRY AND GENETICS OF CELLULOSE DEGRADATION 1988, pages 11 - 30, XP002088719**

**Description**

1. Field of the Invention

The present invention is directed to the use of detergent compositions containing specific cellulase compositions which, when used to wash cotton-containing fabrics, are capable of reducing strength loss as compared to treatment with complete cellulase.

2. State of the Art.

Cellulases are known in the art as enzymes that hydrolyze cellulose ($\beta$-1,4-glucan linkages) thereby resulting in the formation of glucose, cellobiose, cello-oligosaccharides, and the like. While cellulases are produced (expressed) in fungi, bacteria and the like, those produced by fungi have been given the most attention because certain fungi produce a complete cellulase system capable of degrading crystalline forms of cellulose and such cellulases can be readily produced in large quantities via fermentation procedures.

In regard to the above, Wood et al., "Methods in Enzymology," 160. 25, pages 234 at seq. (1988), discloses that certain fungi produce complete cellulase systems which are comprised of several different enzyme classification including those identified as exo-cellobiohydrolases (EC 3.2.1.91) ("CBH"), endoglucanases (EC 3.2.1.4) ("EG"), and $\beta$-glucosidases (EC 3.2.1.21) ("BG"). On the other hand, some fungi are incapable of producing complete cellulase systems. Generally, these systems lack CBH components. See, for instance, Coughlan at al., Biochemistry and Genetics of Cellulose Degradation, Aubert et al., Editor, pages 11 et seq., (Academic Press, 1988); and Wood et al., Methods in Enzymology 160, 25, pages 87 at seq., (Academic Press, New York, 1988).

Likewise, while bacterial cellulases are reported in the literature as containing litlle or no CBH components, there are a few cases where CBH-like components derived from bacterial cellulases have been reported to possess exo-cellobiohydrolase activity.

The fungal cellulase classifications of CBH, EG and BG can be further expanded to include multiple components within each classification. For example, multiple CBHS and EGs have been isolated from a variety of fungal sources including Trichoderma reesei which contains 2 CBHs, i.e., CBH I and CBH II, and at least 3 EGs, i.e., EG I, EG II and EG III.

The complete cellulase system comprising components from each of the CBH, EG and BG classifications is required to efficiently convert crystalline cellulose to glucose. Isolated components are far less effective, if at all, in hydrolyzing crystalline cellulose. Moreover, a synergistic relationship is observed between the cellulase components particularly if they are of different classifications. That is to say the effectiveness of a complete cellulase system is significantly greater than the sum of the contributions from the isolated components of the same classification, In this regard, it is known in the art that the EG components and CBH components synergistically interact to more efficiently degrade cellulose. See, for example, Wood, Biochem. Soc. Trans., 13, pp. 407-410 (1985).

The substrate specificity and mode of action of the different cellulase components varies significantly with classification which may account for the synergy of the combined components. For example, the current accepted mode of cellulase action is that endoglucanase components hydrolyze internal $\beta$-1,4-glucosidic bonds, particularly, in regions of low crystallinity of the cellulose and exo-cellobiohydrolase components hydrolyzes cellobiose from the non-reducing end of cellulose. The action of endoglucanase components greatly facilitates the action of exo-cellobiohydrolases by creating new chain ends which are recognized by exo-cellobiohydrolase components.

$\beta$-Glucosidase components act only on cellooligosaccharides, e.g., cellobiose, to give glucose as the sole product. They are considered an integral part of the cellulase system because they drive the overall reaction to glucose and thereby relieve the inhibitory effects of cellobiose on CBH and EG components.

On the other hand, cellulases are also known in the art to be useful in detergent compositions for the purposes of enhancing the cleaning ability of the composition, for use as a softening agent, and for improving the feel of cotton fabrics, and the like. While the exact mechanism by which cellulase compositions soften garments is not fully understood, softening and color restoration properties of cellulase have been attributed to alkaline endoglucanase components in cellulase compositions. Thus, for instance, International Application Publication No. WO 89/09259 discloses that detergent compositions containing a cellulase composition enriched in a specified alkaline endoglucanase component impart color restoration and improved softening to treated garments as compared to cellulase compositions not enriched in such a component. Additionally, the use of such alkaline endoglucanase components in detergent compositions complements the pH requirements of the detergent composition. The endo-glucanase component of this reference is defined as one exhibiting maximal activity at an alkaline pH of 7.5 to 10 and which has defined activity criteria on carboxymethyl-cellulose and Avicel.

On the other hand, cellulase compositions are known in the art to degrade cotton-containing fabrics (see, for example, Suzuki et al., U.S. Patent No. 4,822,516) which degradation is evidenced by reduced strength loss in the fabric. In turn, such strength loss accounts, in part, for the reluctance to the use of cellulase compositions in commercial detergent applications.

Accordingly, in view of the above, cellulase compositions containing one or more endoglucanase components which also provide reduced strength loss for cotton-containing fabrics as compared to a complete cellulase system would be particularly advantageous for use in detergent compositions.

SUMMARY OF THE INVENTION

It has now been found that fungal cellulase compositions containing one or more endoglucanase type components can be combined in detergent compositions to impart improvements in softening, color retention/restoration and feel to cotton-containing fabrics washed in a wash medium containing such a composition. It has further been found that when such cellulase compositions contain less than about 5 weight percent of CBH I type components, the resulting detergent compositions impart less strength loss to the so-washed cotton-containing fabrics.

In regard to the detergent compositions containing cellulase compostions which are CBHI deficient, CBHI enriched or EGIII enriched, it has been found that it is the amount of cellulase, and not the relative rate of hydrolysis of the specific enzymatic components to produce reducing sugars from cellulose, which imparts the desired detergent properties to cotton-containing fabrics, eg., one or more of improved color restoration, improved softening and improved cleaning to the detergent composition.

Accordingly, in one aspect, the present invention provides the use of a detergent composition for washing a cotton-containing fabric, as defined in the claims.

Preferably, the detergent composition comprises from about 0.05 to about 2 weight percent of said cellulase composition.

In a preferred embodiment, the cellulase composition is free of all exo-cellobiohydrolase components.

In another preferred embodiment, the cellulase composition is derived from a microorganism which has been genetically modified so as to be incapable of producing any CBH I type components and more preferably any CBH type components, i.e., CBH I type and CBH II type components. In even a more preferred embodiment, the cellulase comsition is derived from a microorganism which has been genetically modified so as to be incapable of producing any CBH type components without the expression of any heterologous proteins.

BRIEF DESCRIPTION OF THE DRAWINGS

FIG. 1 is an outline of the construction of pACBHIpyr4.

FIG. 2 illustrates deletion of the T. reesei gene by integration of the larger EcoRI fragment from pACBHIpyr4 at the cbh1 locus on one of the T. reesei chromosomes.

FIG. 3 is an autoradiograph of DNA from T. reesei strain GC69 transformed with EcoRI digested pACBHIpyr4 after Southern blot analysis using a $^{32}$P labelled pACBHIpyr4 as the probe. The sizes of molecular weight markers are shown in kilobase pairs to the left of the Figure.

FIG. 4 is an autoradiograph of DNA from a T. reesei strain GC69 transformed with EcoRI digested pACBHIpyr4 using a $^{32}$P labelled pIntCBHI as the probe. The sizes of molecular weight markers are shown in kilobase pairs to the left of the Figure.

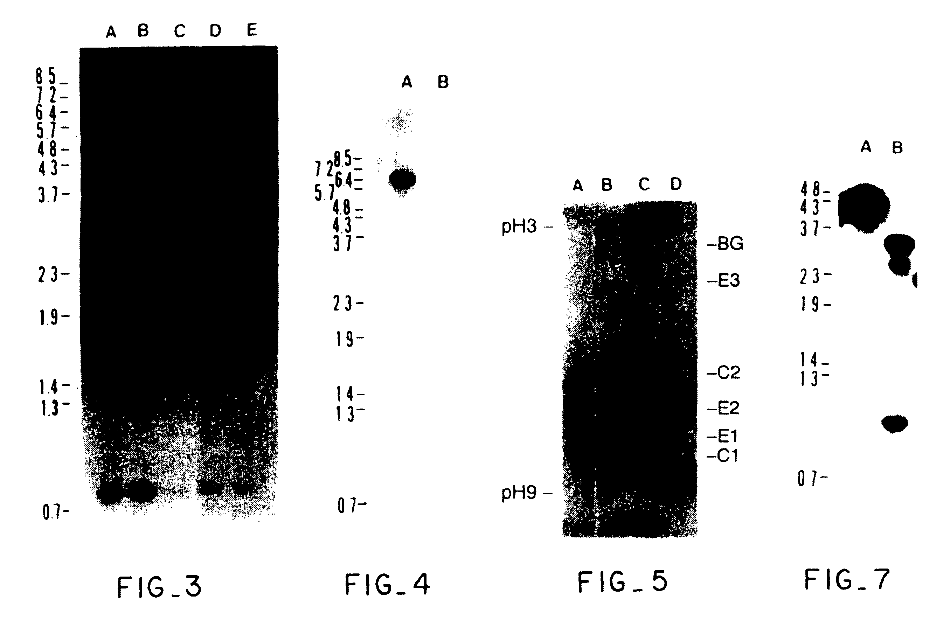

FIG. 5 is an isoelectric focusing gel displaying the proteins secreted by the wild type and by transformed strains of T. reesei. Specifically, in FIG.5, Lane A of the isoelectric focusing gel employs partially purified CBHI from T. reesei; Lane B employs a wild type T. reesei : Lane C employs protein from a T. reesei strain with the cbh1 gene deleted; and Lane D employs protein from a T. reesei strain with the cbh1 and cbh2 genes deleted. In FIG. 5, the right hand side of the figure is marked to indicate the location of the single proteins found in one or more of the secreted proteins. Specifically, BG refers to the β-glucosidase, E1 refers to endoglucanase I, E2 refers to endoglucanase II, E3 refers to endoglucanase III, C1 refers to exocellobiohydrolase I and C2 refers to exocellobiohydrolase II.

FIG. 6A is a representation of the T. reesei cbh2 locus, cloned as a 4.1 kb EcoRI fragment on genomic DNA and FIG. 6B is a representation of the cbh2 gene deletion vector pPACBHII.

FIG. 7 is an autoradiograph of DNA from T. reesei strain P37PΔCBHIPyr26 transformed with EcoRI digested pPΔ-CBHII after Southern blot analysis using a $^{32}$P labelled pPΔCBHII as the probe. The sizes of molecular weight markers are shown in kilobase pairs to the left of the Figure.

FIG. 8 is a diagram of the plasmid pEGIpyr4.

FIG. 9 illustrates the RBB-CMC activity profile of an EG enriched fungal cellulase composition (CBH I and II deleted) derived from Trichoderma reesei over a pH range at 40°C; as well as the activity profile of an enriched EG III cellulase composition derived from Trichoderma reesei over a pH range at 40°C.

FIG. 10 illustrates strength loss results after three wash cycles in a launderometer for cotton-containing fabrics treated with cellulase compositions having varying amounts of CBH components.

FIG. 11 illustrates fiber removal results (based on panel test scores) for cotton-containing fabrics treated with cellulase secreted by a wild type Trichoderma reesei (whole cellulase) at various pHs.

FIG. 12 illustrates fiber removal results (based on panel test scores) for cotton-containing fabrics treated with varying concentrations (in ppm) of cellulase secreted by a wild type Trichoderma reessi and for a cotton fabric treated with cellulase secreted by a strain of Trichoderma reesei genetically engineered so as to be incapable of secreting CBH I and CBH II.

FIG. 13 illustrates the softness panel test results for varying concentrations (in ppm) of an EG enriched cellulase composition derived from a strain of Trichoderma reesei genetically modified so as to be incapable of producing CBH I&II.

FIG. 14 is a diagram of the site specific alterations made in the egl1 and cbh1 genes to create convenient restriction endonuclease cleavage sites. In each case, the upper line shows the original DNA sequence, the changes introduced are shown in the middle line, and the new sequence is shown in the lower line.

FIG. 15 is a diagram of the larger EcoRI fragment which can be obtained from pCEPC1.

FIG. 16 is an autoradiograph of DNA, from an untransformed strain of T. reesei RutC30 and from two transformants obtained by transforming T. reesei with EcoRI digested pCEPC1. The DNA was digested with PstI, a Southern blot was obtained and hybridized with $^{32}$P labelled pUC4K::cbh1. The sizes of marker DNA fragments are shown in kilobase pairs to the left of the Figure.

FIG. 17 is a diagram of the plasmid pEGII::P-1.

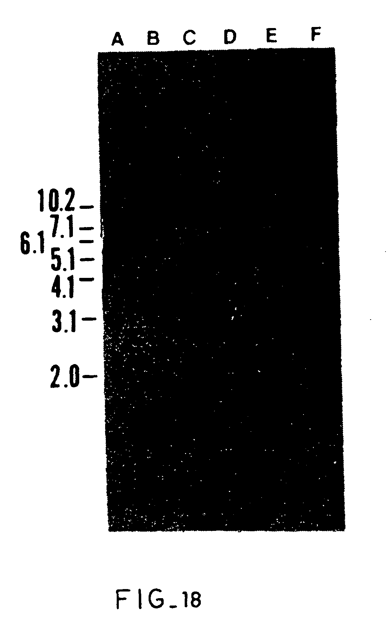

FIG 18. is an autoradiograph of DNA from T. reesei strain P37PΔΔ67P-1 transformed with HindIII and BamHI digested pEGII::P-1. A Southern blot was prepared and the DNA was hybridized with an approximately 4kb PstI fragment of radiolabelled T. reesei DNA containing the egl3 gene. Lanes A, C and E contain DNA from the untransformed strain whereas, Lanes B, D and F contain DNA from the untransformed T. reesei strain. The T. reesei DNA was digested with BglII) in Lanes A and B, with EcoRV in Lanes C and D and with PstI in Lanes E and F. The size of marker DNA fragments are shown in kilobase pairs to the left of the Figure.

FIG. 19 is a diagram of the plasmid pPΔEGI-1.

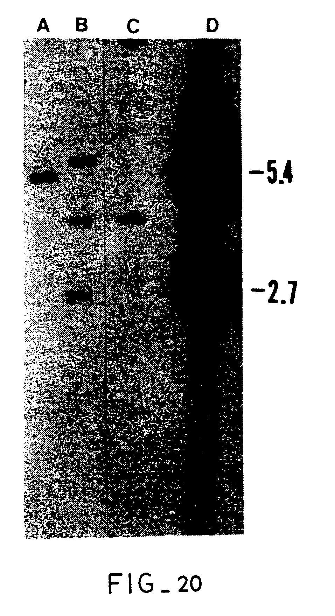

FIG. 20 is an autoradiograph of a Southern blot of DNA isolated from transformants of strain GC69 obtained with HindIII digested pΔEGIpyr-3. The pattern of hybridisation with the probe, radiolabelled pΔEGIpyr-3, expected for an untransformed strain is shown in Lane C. Lane A shows the pattern expected for a transformant in which the egl1 gene has been disrupted and Lane B shows a transformant in which pΔEGIpyr-3 DNA has integrated into the genome but without disrupting the egl1 gene. Lane D contains pΔEGIpyr-3 digested with HindIII to provide appropriate size markers. The sizes of marker DNA fragments are shown in kilobase pairs to the right of the figure.

DETAILED DESCRIPTION OF THE INVENTION

As noted above, the present invention generally relates to the use of detergent compositions containing fungal cellulase compositions as defined in the claims. When used in wash media having acidic, neutral or alkaline pH's, such detergent compositions impart improvements in softening, color retention/restoration, and feel to cotton-containing fabrics washed in such media. Moreover, because of the small amount of CBH I type components present in the cellulase composition, these compositions also provide reduced strength loss as compared to those compositions containing greater amounts of CBH I type components.

However, prior to discussing this invention in detail, the following terms will first be defined.

The term "fungal cellulase" refers to an enzyme composition derived from fungal sources or microorganisms genetically modified so as to incorporate and express all or part of the cellulase genes obtained from a fungal source. Fungal cellulases act on cellulose or one or more of its degradation products to hydrolyze cellulose and give primary products, glucose and cellobiose. Fungal cellulases are distinguished from cellulases produced from non-fungal sources including microorganisms such as actinomycetes, gliding bacteria (myxobacteria) and true bacteria. Fungi capable of producing cellulases useful in preparing cellulase compositions used in the detergent compositions described herein are disclosed in British Patent No. 2 094 826A, the disclosure of which is incorporated herein by reference.

Most fungal cellulases generally have their optimum activity in the acidic or neutral pH range although some fungal cellulases are known to possess significant activity under neutral and slightly alkaline conditions, i.e., for example, cellulase derived from Humicola insolens is known to have activity in neutral to slightly alkaline conditions.

Fungal cellulases are known to be comprised of several enzyme classifications having different substrate specificity, enzymatic action patterns, and the like. Additionally, enzyme components within each classification can exhibit different molecular weights, different degrees of glycosylation, different isoelectric points, different substrate specificity, different enzymatic action patterns, etc. For example, fungal cellulases can contain cellulase classifications which include endoglucanases (EGs), exo-cellobiohydrolases (CBHs), β-glucosidases (BGs), etc. On the other hand, while bacterial cellulases are reported in the literature as containing little or no CBH components, there are a few cases where CBH-like components derived from bacterial cellulases have been reported to possess exo-cellobiohydrolase activity.

A fungal cellulase composition produced by a naturally occurring fungal source and which comprises one or more CBH and EG components wherein each of these components is found at the ratio produced by the fungal source is sometimes referred to herein as a "complete fungal cellulase system" or a "complete fungal cellulase composition" to distinguish it from the classifications and components of cellulase isolated therefrom, from incomplete cellulase compositions produced by bacteria and some fungi, or from a cellulase composition obtained from a microorganism genetically modified so as to overproduce, underproduce or not produce one or more of the CBH and/or EG components of cellulase.

The fermentation procedures for culturing fungi for production of cellulase are known per se in the art. For example, cellulase systems can be produced either by solid or submerged culture, including batch, fed-batch and continuous-flow processes. The collection and purification of the cellulase systems from the fermentation broth can also be effected by procedures known per se in the art.

"Endoglucanase ("EG") type components" refer to all of those fungal cellulase components or combination of components which exhibit detergent activity properties similar to the endoglucanase components of Trichoderma Trichoderma reesei In this regard, the endoglucanase components of Trichoderma reesei (specifically, EG I, EG II, EG III, and the like either alone or in combination) impart softening, color retention/restoration and improved feel to cotton-containing fabrics when these components are incorporated into a wash medium and the fabric is treated with this medium. Accordingly, endoglucanase type components are those fungal cellulase components which impart softening, color retention/restoration and improved feel to cotton garments when these components are incorporated into a wash medium. In a preferred embodiment, the endoglucanase type components employed in the detergent compositions of this invention also impart less strength loss to cotton-containing fabrics as compared to strength loss arising from the complete cellulase system derived from Trichoderma reesei.

Such endoglucanase type components may not include components classified as endoglucanases using traditional biochemical activity tests. For example, such traditional activity tests are based on the ability of the component (a) to hydrolyze soluble cellulose derivatives such as carboxymethylcellulose (CMC),thereby reducing the viscosity of CMC containing solutions, (b) to readily hydrolyze hydrated forms of cellulose such as phosphoric acid swollen cellulose (e.g., Walseth cellulose) and hydrolyze less readily the more highly crystalline forms of cellulose (e.g., Avicel, Solkafloc, etc.). In contrast, it is believed that not all endoglucanase components, as defined by such activity tests, will provide improved softness, feel and color retention/restoration. Accordingly, it is more accurate for the purposes herein to define endo-glucanase type components as those components of fungal cellulase which possess similar properties in detergent compositions as possessed by the endoglucanase components of Trichoderma reesei.

Fungal cellulases can contain more than one EG type component. The different components generally have different isoelectric points, different molecular weights, different degrees of glycosylation, different substrate specificity. different enzymatic action patterns, etc. The different isoelectric points of the components allow for their separation via ion exchange chromatography and the like. In fact, the isolation of components from different fungal sources is known in the art. See, for example, Bjork et al., U.S. Serial No. 07/422,814, Schulein et al., International Application WO 89/09259, Wood et al., Biochemistry and Genetics of Cellulose Degradation, pp. 31 to 52 (1988); Wood et al., Carbohydrate Research, Vol. 190, pp. 279 to 297 (1989); Schulein, Methods in Enzymology, Vol. 160, pp. 234 to 242 (1988) ; and the like. The entire disclosure of each of these references is incorporated herein by reference.

In general, it is contemplated that combinations of EG type components may give a synergistic response in improving softening, color retention/restoration and feel as compared to a single EG type component. On the other hand, a single EG type component may be more stable or have a broader spectrum of activity over a range of pHs. Accordingly, the EG type components employed in this invention can be either a single EG type component or a combination of two or more EG type components. When a combination of components is employed, the EG type components may be derived from the same or different fungal sources.

"Exo-cellobiohydrolase type ("CBH typed components" refer to those fungal cellulase components which exhibit detergent activity properties similar to CBH I and/or CBH II components of Trichoderma reesei. In this regard, when used in the absence of EG type components (as defined above), the CBH I and CBH II components of Trichoderma reesei alone do not impart significant color retention/re-storation and improved feel to the so-treated cotton-containing fabrics. Additionally, when used in combination with EG type components, the CBH I component of Trichoderma reesei imparts enhanced strength loss and an incremental cleaning effect to cotton-containing fabrics.

Accordingly CBH I type components and CBH II type components refer to those fungal cellulase components which exhibit detergent activity properties similar to CBH I and CBH II components of Trichoderma reesei. respectively. As noted above, for CBH I type components, this includes the properties of enhancing strength loss of cotton-containing fabrics and/or imparting an incremental cleaning benefit when used in the presence of EG type components. In a preferred embodiment, the CBH I components also impart an incremental softening benefit when used in the presence of EG type components.

Such exo-cellobiohydrolase type components may include components not traditionally classified as exo-cellobio-hydrolases using activity tests such as those used to characterize CBH I and CBH II from Trichoderma reesei. For example, using such traditional classification tests, such components are: (a) competitively inhibited by cellobiose ($K_i$ approximately 1 mM); (b) unable to hydrolyze to any significant degree substituted celluloses, such as carboxy-methylcellulose, etc., and (c) able to hydrolyze phosphoric acid swollen cellulose and to a lesser degree highly crystalline cellulose. In contrast, it is believed that some fungal cellulase components which are characterized as CBH components by such activity tests, will provide improved softness, feel and color retention/restoration to cotton-containing fabrics when these components are used alone in detergent compositions. Accordingly, it is believed to be more accurate for the purposes herein to define such exo-cellobiohydrolases as EG type components because these components possess similar functional properties in detergent compositions as possessed by the endoglucanase components of Trichoderma reesei.

Fungal cellulases enriched in EG type components can be obtained by purification techniques. Speafically, the complete cellulase system can be purified into sub-stantially pure components by recognized separation techniques well published in the literature, including ion exchange chromatography at a suitable pH, affinity chromatography, size exclusion and the like. For example, in ion exchange chromatography (usually anion exchange chromatography), it is possible to separate the cellulase components by eluting with a pH gradient, or a salt gradient, or both a pH and a salt gradient

It is also contemplated that mixtures of cellulase components having the requisite ratio of EG type components to CBH I type cellulase components could be prepared by means other than isolation and recombination of the components. In this regard, it may be possible to modify the fermentation conditions for a natural microorganism in order to give relatively high ratios of EG to CBH components. Likewise, recombinant techniques can after the relative ratio of EG components to CBH components so as to produce a mixture of cellulase components having a relatively high ratio of EG components to CBH components or which can produce one or more EG components free of all CBH components.

In regard to the above, a preferred method for the preparation of cellulase compositions enriched in EG type components is by genetically modifying a microorganism so as to be incapable of producing one or more CBH type components which methods do not produce any heterologous protein. Likewise, it is also possible to genetically modify a microorganism so as to overproduce one or more EG type components. For example, U.S. Serial No. 07/593,919, filed October 5, 1990 and which is incorporated herein by reference in its entirety, discloses methods for genetically engineering Trichoderma reesei so as to be incapable of producing one or more CBH components and/or overproducing one or more EG components. Moreover, the methods of that application create Trichoderma reesei strains which do not produce any heterologous proteins. Likewise, Miller et al., "Direct and Indirect Gene Replacement in Aspergillus nidulans", Molecular and Cellular Biology, p. 1714-1721 (1985) discloses methods for deleting genes in Aspergillus nidulans by DNA mediated transformation using a linear fragment of homologous DNA. The methods of Miller et al., would achieve gene deletion without producing any heterologous proteins.

In view of the above, the deletion of the genes responsible for producing either CBH I type or CBH II type cellulase components would have the effect of enriching the amount of EG type components present in the cellulase composition. Likewise, the deletion of those genes responsible for producing CBH I and **II** type components would result in a cellulase composition free of CBH type components.

It is still further contemplated that fungal cellulase compositions can be used herein from fungal sources which produce an incomplete fungal cellulase composition. For example it is known that certain fungi produce cellulase compositions free of CBH components. See, for example, Coughlan et al., Biochemistry and Genetics of Cellulose Degradation, Aubert et al. Editors, pp. 11-30 (Academic Press, 1988), disclose that brown rot fungi do not apparently produce CBH components, but it may be possible that one or more of these components are CBH I type components. On the other hand, if a sufficient amount of the endoglucanases produced by such fungi are, for the purposes herein, EG type components, then it would be possible to use such cellulase in the practice of this invention without enrichment to obtain the necessary ratio of EG type components to CBH I type components.

Additionally, a requisite amount of one or more CBH type components purified by conventional procedures can be added to a cellulase composition produced from a micro-organism genetically engineered so as to be incapable of producing CBH type components so as to achieve a specified ratio of EG type components to one or more CBH type components, i.e., a cellulase composition free of all CBH type components so as to be enriched in EG type components can be formulated to contain 1 weight percent of a CBH I type component merely by adding this amount of a purified CBH I type component to the cellulase composition.

"β-Glucosidase (BG) components" refer to those components of cellulase which exhibit BG activity; that is to say that such components will act from the non-reducing end of cellobiose and other soluble cellooligosaccharides ("cellobiose") and give glucose as the sole product. BG components do not adsorb onto or react with cellulose polymers. Furthermore, such BG components are competitively inhibited by glucose ($K_i$ approximately 1 mM). While in a strict sense, BG components are not literally cellulases because they cannot degrade cellulose, such BG components are included within the definition of the cellulase system because these enzymes facilitate the overall degradation of cellulose by further degrading the inhibitory cellulose degradation products (particularly cellobiose) produced by the combined action of CBH components and EG components. Without the presence of BG components, moderate or little hydrolysis of crystalline cellulose will occur. BG components are often characterized on aryl substrates such as p-nitrophenol B-D-glucoside (PNPG) and thus are often called aryl-glucosidases. It should be noted that not all aryl-glucosidases are BG components, in that some do not hydrolyze cellobiose.

It is contemplated that the presence or absence of BG components in the cellulase composition can be used to regulate the activity of the CBH components. Specifically, because cellobiose is produced during cellulose degradation by CBH components, and because high concentrations of cellobiose are known to inhibit CBH activity, and further because such cellobiose is hydrolyzed to glucose by BG components, the absence of BG components in the cellulase composition will "turn-off" CBH activity when the concentration of cellobiose reaches inhibitory levels. It is also contemplated that one or more additives (e.g., cellobiose, glucose, etc.) can be added to the cellulase composition to effectively "turn-off", directly or indirectly, some or all of the CBH I type activity as well as other CBH activity. When such additives are employed, the resulting composition is considered to be a composition suitable for use in this invention if the amount of additive employed is sufficient to lower the CBH I type activity to levels equal to or less than the CBH I type activity levels achieved by using the cellulase compositions described herein.

On the other hand, a cellulase composition containing added amounts of BG components may increase overall hydrolysis of cellulose if the level of cellobiose generated by the CBH components becomes restrictive of such overall hydrolysis in the absence of added BG components.

Methods to either increase or decrease the amount of BG components in the cellulase composition are disclosed in U.S. Serial No. 07/625,140, filed December 10, 1990, as attorney docket no. 010055-056 and entitled "SAC-CHARIFICATION OF CELLULOSE BY CLONING AND AMPLIFICATION OF THE β-GLUCOSIDASE GENE OF TRICHODERMA REESEI", which application is incorporated herein by reference.

Fungal cellulases can contain more than one BG component. The different components generally have different isoelectric points which allow for their separation via ion exchange chromatography and the like. Either a single BG component or a combination of BG components can be employed.

When employed in the detergent composition, the BG component is generally added in an amount sufficient to prevent inhibition of the CBH and EG components and particularly, CBH I type cellulase components, by cellobiose. The amount of BG component added depends upon the amount of cellobiose produced in the detergent wash which can be readily determined by the skilled artisan. However, when employed, the weight percent of BG component relative to CBH type components in the cellulase composition is preferably from about 0.2 to about 10 weight percent, and more preferably, from about 0.5 to about 5 weight percent.

Preferred fungal cellulases for use in preparing the fungal cellulase compositions used in this invention are those obtained from <u>Trichoderma</u> <u>reesei,</u> <u>Trichoderma</u> <u>Trichoderma</u> <u>koningii,</u> <u>Pencillum</u> <u>sp</u>., <u>Humicola</u> <u>insoles,</u> and the like. Certain fungal cellulases are commercially available, i.e., CELLUCAST (available from Novo Industry, Copenhagen, Denmark), RAPIDASE (available from Gist Brocades, N.V., Delft, Holland), CYTOLASE 123 (available from Genencor International, South San Francisco, California) and the like. Other fungal cellulases can be readily isolated by art recognized fermentation and isolation procedures.

The term "cotton-containing fabric" refers to sewn or unsewn fabrics made of pure cotton or cotton blends including cotton woven fabrics, cotton knits, cotton denims, and the like. When cotton blends are employed, the amount of cotton in the fabric should be at least about 40 percent by weight cotton; preferably, more than about 60 percent by weight cotton; and most preferably, more than about 75 percent by weight cotton. When employed as blends, the companion material employed in the fabric can include one or more non-cotton fibers including synthetic fibers such as polyamide fibers (for example, nylon 6 and nylon 66), acrylic fibers (for example, polyacrylonitrile fibers), polyester fibers (for example, polyethylene terephthalate), polyvinyl alcohol fibers (for example, Vinylon), polyvinyl chloride fibers, polyvinylidene chloride fibers, polyurethane fibers, polyurea fibers and aramid fibers. It is contemplated that regenerated cellulose, such as rayon, could be used as a substitute for cotton in cotton-containing fabrics.

The term "surface active agent or surfactant" refers to anionic, non-ionic and ampholytic surfactants well known for their use in detergent compositions.

The term "wash medium" refers to an aqueous wash solution prepared by adding a requisite amount of a detergent (surfactant) composition to water. The wash medium generally contains a cleaning effective amount of the detergent.

The wash medium is defined as an "acidic wash medium" if the pH of the medium is from about 4 to less than about 7. The wash medium is defined as a "neutral wash medium" if the pH of the medium is about 7. The wash medium is defined as an "alkaline wash medium" if the pH of the medium is from above 7 to about 10. Preferably, the alkaline wash medium will have a pH of from above 7 to about 9 and, even more preferably, from above 7 to about 8. The present invention is directed to the discovery that EG type components can be used in detergent compositions to effect softening as well as color retention/restoration and feel of cotton-containing fabrics regardless of whether such compositions are employed in an acidic, neutral or alkaline wash medium. However, because detergent compositions are generally employed in alkaline conditions, the cellulase composition employed is preferably one which possesses some activity under such conditions. Preferred cellulase compositions include those derived from T. reesei and Humicola insolens. Cellulase compositions from Humicola insolens are known in the art to retain significant activity under alkaline conditions.

Although the presence of EG type components are necessary to effect color retention/restoration, softening and improved feel, specified mixtures of EG type components and CBH type components (including CBH I type components) also show the same benefits or even some incremental benefits. Specifically, while the use of EG components will result in some cleaning benefit, an incremental cleaning benefit is observed for cotton-containing fabrics washed with a detergent composition containing a cellulase composition containing one or more EG type components and which contains CBH I type cellulase components. Additionally, while the use of EG components will effect softening, an incremental softening benefit may be achieved by incorporating some CBH I type components with the EG type components.

On the other hand, the presence of significant amounts of CBH I type components in combination with the EG type components results in enhanced strength loss to cotton-containing fabrics compared to cellulase compositions which are either free of CBH I type components or contain reduced amounts of CBH I type components. Accordingly, in order to minimize the strength loss characteristics of cellulase containing detergent compositions, the detergent composi-tions described herein employ cellulase composi-tions containing one or more EG type components and less than about 5 weight percent, and preferably less than about 2 weight percent, of CBH I type components. Detergent compositions containing such cellulase compositions provide the desired enhancements to the cotton-containing fabrics while providing for reduced strength loss as compared to detergent compositions containing a cellulase composition which has greater amounts of CBH I type components.

Further in regard to the above, the selection of the specific cellulase composition for use in the detergent composition of this invention is made by balancing the desire for an incremental cleaning benefit achieved by the presence of some CBH I type components against that for strength loss resistance which dictates the use of minimal or no CBH I type components. That is to say that if the primary emphasis for incorporating cellulase into the detergent composition is for improved softness, color retention/restoration, and improved feel of cotton-containing fabrics with the minimum possible strength loss to the cotton-containing fabric, then the cellulase employed in the detergent composition should preferably comprise one or more EG type components free of all CBH I type components.

On the other hand, if the primary emphasis for incorporating cellulase into the detergent composition is for color retention/restoration, improved softness and improved feel of cotton-containing fabrics, in conjunction with an incremental cleaning benefit, then the cellulase employed in the detergent composition should contain a cellulase comprising one or more EG type components as well as some CBH I type component (albeit less than about 5 weight percent, preferably less than about 2 weight percent, based on the total protein weight of the cellulase composition).

In regard to the above, the amount of cellulase generally employed in the detergent compositions described herein is an amount sufficient to impart color retention/restoration and softness to the cotton garments. Preferably, the cellulase compositions are employed from about 0.01 weight percent to about 5 weight percent relative to the weight of the total detergent composition. More preferably, the cellulase compositions are employed from about 0.05 weight percent to about 2 weight percent relative to the weight of the total detergent composition. The specific concentration of cellulase employed in the detergent composition is selected so that upon dilution into a wash medium, the concentration of EG type components will range from about 0.5 to about 500 ppm, and preferably from about 2 to about 100 ppm. The specific amount of cellulase employed in the detergent composition will depend on the amount of EG type components in the cellulase compositions as well as the extent the detergent composition will be diluted upon addition to water to form a wash medium. These factors are readily ascertained by the skilled artisan.

Preferably, the cellulase compositions employed in the detergent compositions described herein contain at least about 20 weight percent of endoglucanase type components based on the total weight of protein in the cellulase composition, more preferably, at least about 50 weight percent, and even more preferably, at least about 70 weight percent, and most preferably, at least about 90 weight percent of endoglucanase type components based on the total weight of protein in the cellulase composition.

At lower cellulase concentrations (i.e., concentrations of EG type components of less than about 5 ppm in the wash medium), softness, color retention/restoration and improved feel achieved by use of the detergent compositions of this invention is more evident over repeated washings. At higher concentrations (i.e., concentrations of EG type components of about 5 ppm and above in the wash medium), the improvements can become noticeable in a single wash.

One of the important aspects of the present invention is that by tailoring the cellulase composition to contain one or more EG components and to contain minimal amounts of CBH I type components, it is possible to achieve the desired effects of softening, color retention/restoration and improved feel while reducing strength loss to the cotton-containing fabric.

Additionally, the use of such tailored cellulase compositions permits the use of lower concentrations of cellulase in the detergent composition. In turn, the use of lower concentrations of cellulase in the detergent compositions should lead to improved consumer safety.

The cellulase composition as described above can be added to the detergent composition either in a liquid diluent, in granules, in emulsions, in gels, in pastes, and the like. Such forms are well known to the skilled artisan. When a solid detergent composition is employed, the cellulase composition is preferably formulated as granules. Preferably, the granules can be formulated so as to contain a cellulase protecting agent. See, for instance, U.S. Serial No. 07/642,669 filed January 17, 1991 as Attorney Docket No. 010055-073 and entitled "GRANULES CONTAINING BOTH AN ENZYME AND AN ENZYME PROTECTING AGENT AND DETERGENT COMPOSITIONS CONTAINING SUCH GRANULES" which application is incorporated herein by reference in its entirety. Likewise, the granule can be formulated so as to contain materials to reduce the rate of dissolution of the granule into the wash medium. Such materials and granules are disclosed in U.S. Serial No. 07/642,596 filed on January 17, 1991 as Attorney Docket No. GCS-171-US1 and entitled "GRANULAR COMPOSITIONS" which application is incorporated herein by reference in its entirety.

The detergent compositions described herein employ a surface active agent, i.e., surfactant, including anionic, non-ionic and ampholytic surfactants well known for their use in detergent compositions

Suitable anionic surfactants for use in the detergent composition described herein include linear or branched alkylbenzenesulfonates; alkyl or alkenyl ether sulfates having linear or branched alkyl groups or alkenyl groups; alkyl or alkenyl sulfates; olefinsulfonates; alkane-sulfonates and the like. Suitable counter ions for anionic surfactants include alkali metal ions such as sodium and potassium; alkaline earth metal ions such as calcium and magnesium; ammonium ion; and alkanolamines having 1 to 3 alkanol groups of carbon number 2 or 3.

Ampholytic surfactants include quaternary ammonium salt sulfonates, betaine-type ampholytic surfactants, and the like. Such ampholytic surfactants have both the positive and negative charged groups in the same molecule. Nonionic surfactants generally comprise polyoxy-alkylene ethers, as well as higher fatty acid alkanolamides or alkylene oxide adduct thereof, fatty add glycerine monoesters, and the like.

Suitable surfactants for use in this invention are disclosed in British Patent Application No. 2 094 826 A, the disclosure of which is incorporated herein by reference.

Mixtures of such surfactants can also be used.

The surfactant or a mixture of surfactants is generally employed in the detergent compositions of this invention described herein in an amount from about 1 weight percent to about 95 weight percent of the total detergent composition and preferably from about 5 weight percent to about 45 weight percent of the total detergent composition. Upon dilution in the wash medium, the surfactant concentration is generally about 500 ppm or more; and preferably, from about 1000 ppm to 15,000 ppm.

In addition to the cellulase composition and the surfactant(s), the detergent compositions described herein can optionally contain one or more of the following components:

Hydrolases except cellulase

Such hydrolases include carboxylate ester hydrolase, thioester hydrolase, phosphate monoester hydrolase, and phosphate diester hydrolase which act on the ester bond; glycoside hydrolase which acts on glycosyl compounds; an enzyme that hydrolyzes N-glycosyl compounds; thioether hydrolase which acts on the ether bond; and $\alpha$-aminoacyl-peptide hydrolase, peptidyl-amino acid hydrolase, acyl-amino acid hydrolase, dipeptide hydrolase, and peptidyl-peptide hydrolase which act on the peptide bond. Preferable among them are carboxylate ester hydrolase, glycoside hydrolase, and peptidyl-peptide hydrolase. Suitable hydrolases include (1) proteases belonging to peptidyl-peptide hydrolase such as pepsin, pepsin B, rennin, trypsin, chymotrypsin A, chymotrypsin B, elastase, enterokinase, cathepsin C, papain, chymopapain, ficin, thrombin, fibrinolysin, renin, subtilisin, aspergillopepti-dase A, collagenase, clostridi-opeptidase B, kallikrein, gastrisin, cathepsin D., bromelin, keratinase, chymotrypsin C, pepsin C, aspergillopeptidase B, urokinase, carboxy-peptidase A and B, and aminopeptidase; (2) glycoside hydrolases (cellulase which is an essential ingredient is excluded from this group) α-amylase, β-amylase, gluco amylase, invertase, lysozyme, pectinase, chitinase, and dextranase. Preferably among them are α-amylase and β-amylase. They function in acid to neutral systems, but one which is obtained from bacteria exhibits high activity in an alkaline system; (3) carboxylate ester hydrolase including carboxyl esterase, lipase, pectin esterase, and chlorophyllase. Especially effective among them is lipase.

Trade names of commercial products and producers are as follows: "Alkalase", "Esperase", "Savinase", "AMG", "BAN", "Fungamill", "Sweetzyme", "Thermamyl" (Novo Industry, Copenhagen, Denmark); "Malcsatase", "High-alkaline protease", "Amylase THC", "Lipase" (Gist Brocades, N.V, Delft, Holland); "Protease B-400", "Protease B-4000", "Protease AP", "Protease AP 2100" (Schweizerische Ferment A.G., Basel, Switzerland); "CRD Protease" (Monsanto Company, St. Louis, Missouri); "Piocase" (Piopin Corporation, Monticello, Illinois); "Pronase P", "Pronase AS", "Pronase AF" (Kaken Chemical Co., Ltd., Japan); "Lapidase P-2000" (Lapidas, Secran, France); protease products (Tyler standard sieve, 100% pass 16 mesh and 100% on 150 mesh) (Clington Corn Products, Division of Standard Brands Corp., New York); "Takamine", "Bromelain 1:10", "HT Protease 200", "Enzyme L-W" (obtained from fungi, not from bacteria) (Miles Chemical Company, Elkhart, Ind.); "Rhozyme P-11 Conc.", "Pectinol", "Lipase B", "Rhozyme PF", "Rhozyme J-25" (Rohm & Haas, Genencor, South San Francisco, CA); "Ambrozyme 200" (Jack Wolf & Co., Ltd., Subsidiary of Nopco Chemical Company, Newark, N.J.); "ATP 40", "ATP 120", "ATP 160" (Lapides, Secran, France): "Oripase" (Nagase & Co., Ltd., Japan).

The hydrolase other than cellulase is incorporated into the detergent composition as much as required according to the purpose. It should preferably be incorporated in an amount of 0.001 to 5 weight percent, and more preferably 0.02 to 3 weight percent, in terms of purified one. This enzyme should be used in the form of granules made of crude enzyme alone or in combination with other components in the detergent composition. Granules of crude enzyme are used in such an amount that the purified enzyme is 0.001 to 50 weight percent in the granules. The granules are used in an amount of 0.002 to 20 and preferably 0.1 to 10 weight percent. As with cellulases, these granules can be formulated so as to contain an enzyme protecting agent and a dissolution retardant material.

Cationic surfactants and long-chain fatty acid salts

Such cationic surfactants and long-chain fatty acid salts include saturated or unsaturated fatty acid salts, alkyl or alkenyl ether carboxylic acid salts, α-sulfofatty add salts or esters, amino acid-type surfactants, phosphate ester surfactants, quaternary ammonium salts including those having 3 to 4 alkyl substituents and up to 1 phenyl substituted alkyl substituents. Suitable cationic surfactants and long-chain fatty acid salts are disclosed in British Patent Application No. 2 094 826 A, the disclosure of which is incorporated herein by reference. The composition may contain from about 1 to about 20 weight percent of such cationic surfactants and long-chain fatty acid salts.

Builders

A. Divalent sequestering agents.

The composition may contain from about 0 to about 50 weight percent of one or more builder components selected from the group consisting of alkali metal salts and alkanolamine salts of the following compounds: phosphates, phosphonates, phosphonocarboxylates, salts of amino acids, aminopolyacetates high molecular electrolytes, non-dissociating polymers, salts of dicarboxylic acids, and aluminosilicate salts. Suitable divalent sequestering gents are disclosed in British Patent Application No. 2 094 826 A, the disclosure of which is incorporated herein by reference.

B. Alkalis or inorganic electrolytes

The composition may contain from about 1 to about 50 weight percent, preferably from about 5 to about 30 weight percent, based on the composition of one or more alkali metal salts of the following compounds as the alkalis or inorganic electrolytes: silicates, carbonates and sulfates as well as organic alkalis such as triethanolamine, diethanolamine, monoethanolamine and triisopropanolamine.

Antiredepositon agents

The composition may contain from about 0.1 to about 5 weight percent of one or more of the following compounds as antiredeposition agents: polyethylene glycol, polyvinyl alcohol, polyvinylpyrrolidone and carboxymethylcellulose.
Among them, a combination of carboxymethylcellulose or/and polyethylene glycol with the cellulase composition described herein provides for an especially useful dirt removing composition.

### Bleaching agents

The use of the cellulase described herein in combination with a bleaching agent such as sodium percarbonate, sodium perborate, sodium sulfate/hydrogen peroxide adduct and sodium chloride/hydrogen peroxide adduct or/and a photo-sensitive bleaching dye such as zinc or aluminum salt of sulfonated phthalocyanine further improves the deterging effects.

### Bluing agents and fluorescent dyes

Various bluing agents and fluorescent dyes may be incorporated in the composition, if necessary. Suitable bluing agents and fluorescent dyes are disclosed in British Patent Application No. 2 094 826 A, the disclosure of which is incorporated herein by reference.

### Caking inhibitors

The following caking inhibitors may be incorporated in the powdery detergent: p-toluenesulfonic acid salts, xylenesulfonic acid salts, acetic acid salts, sulfosuccinic acid salts, talc, finely pulverized silica, clay, calcium silicate (such as Micro-Cell of Johns Manville Co.), calcium carbonate and magnesium oxide.

### Masking agents for factors inhibiting the cellulase activity

The cellulase composition described herein are deactivated in some cases in the presence of copper, zinc, chromium, mercury, lead, manganese or silver ions or their compounds. Various metal chelating agents and metal-precipitating agents are effective against these inhibitors. They include, for example, divalent metal ion sequestering agents as listed in the above item with reference to optional additives as well as magnesium silicate and magnesium sulfate.

Cellobiose, glucose and gluconolactone act sometimes as the inhibitors. It is preferred to avoid the co-presence of these saccharides with the cellulase as far as possible. In case the co-presence in unavoidable, it is necessary to avoid the direct contact of the saccharides with the cellulase by, for example, coating them.

Long-chain-fatty add salts and cationic surfactants act as the inhibitors in some cases. However, the co-presence of these substances with the cellulase is allowable if the direct contact of them is prevented by some means such as tableting or coating.

The above-mentioned masking agents and methods may be employed, if necessary, in the present invention.

### Cellulase-activators

The activators vary depending on variety of the cellulases. In the presence of proteins, cobalt and its salts, magnesium and its salts, and calcium and its salts, potassium and its salts, sodium and its salts or mono-saccharides such as mannose and xylose, the cellulases are activated and their deterging powers are improved remarkably.

### Antioxidants

The antioxidants include, for example, tert-butyl-hydroxytoluene, 4,4'-butylidenebis(6-tert-butyl-3-methyl-phenol), 2,2'-butylidenebis(6-tertfiutyl-4-methylphenol), monostyrenated cresol, distyrenated cresol, monostyrenated phenol, distyrenated phenol and 1,1-bis(4-hydroxy-phenyl)cyclohexane.

### Solubilizers

The solubilizers include, for example, lower alcohols such as ethanol, benzenesulfonate salts, lower alkyl-benzenesulfonate salts such as p-toluenesuffonate salts, glycols such as propylene glycol, acetylbenzenesulfonate salts, acetamides, pyridinedicarboxylic acid amides, benzoate salts and urea.

The detergent composition described herein can be used in a broad pH range of from acidic to alkaline pH. In a preferred embodiment, the detergent composition can be used in alkaline detergent wash media and more preferably, alkaline detergent wash media having a pH of from above 7 to no more than about 8.

Aside from the above ingredients, perfumes, buffers, preservatives, dyes and the like can be used, if desired, with the detergent compositions of this invention. Such components are conventionally employed in amounts heretofore used in the art.

When a detergent base used in the present invention is in the form of a powder, it may be one which is prepared by any known preparation methods including a spray-drying method and a granulation method. The detergent base obtained particularly by the spray-drying method and/or spray-drying granulation method are preferred. The detergent base obtained by the spray-drying method is not restricted with respect to preparation conditions. The detergent base obtained by the spray-drying method is hollow granules which are obtained by spraying an aqueous slurry of heat-resistant ingredients, such as surface active agents and builders, into a hot space. The granules have a size of from 50 to 2000 micrometers. After the spray-drying, perfumes, enzymes, bleaching agents, inorganic alkaline builders may be added. With a highly dense, granular detergent base obtained such as by the spray-drying-granulabon method, various ingredients may also be added after the preparation of the base.

When the detergent base is a liquid, it may be either a homogeneous solution or an inhomogeneous dispersion. For removing the decomposition of carboxymethylcellulose by the cellulase in the detergent, it is desirable that carboxy-methytcellulose is granulated or coated before the incorporation in the composition.

The detergent compositions described herein are used in industrial and household uses at temperatures and liquor ratios conventionally employed in these environments.

In addition to their use in laundry detergents, the cellulase compositions described herein can additionally be used in a pre-washing step in the appropriate solution at an intermediate pH where sufficient activity exists to provide desired improvements in color retention/restoration, softening and feel. When the cellulase composition is employed in a pre-soak (e.g., pre-wash) composition, either as a liquid, spray, gel or paste composition, the cellulase composition is generally employed from about 0.01 to about 20 weight percent based on the total weight of the pre-soak composition. In such compositions, a surfactant may optionally be employed and when employed, is generally present at a concentration of from about 0.01 to about 20 weight percent based on the total weight of the pre-soak. The remainder of the composition comprises conventional components used in the pre-soak, i.e., diluent, buffers, other enzymes (proteases), and the like at their conventional concentrations. Accordingly, such pre-soak compositions comprise from about 0 to about 20 weight percent of a surfactant and from about 0.01 to about 20 weight percent of a cellulase comprising one or more EG type components and less than about 5 weight percent CBH I type components.

Also, it is contemplated that the cellulase compositions described herein can also be used in home use as a stand alone composition suitable for restoring color to faded fabrics. See, for example, U.S. Patent No. 4,738,682, which is incorporated herein by reference in its entirety.

The following examples are offered to illustrate the present invention and should not be construed in any way as limiting the scope of this invention.

EXAMPLES

Examples 1-12 and 20-28 demonstrate the preparation of Trichoderma reesei genetically engineered so as to be incapable of producing one or more cellulase components or so as to overproduce specific cellulase components.

Example 1

Selection for pyr4 derivatives of Trichoderma reesei

The pyr4 gene encodes orotidine-5'-monophosphate decarboxylase, an enzyme required for the biosynthesis of uridine. The toxic inhibitor 5-fluoroorotic acid (FOA) is incorporated into uridine by wild-type cells and thus poisons the cells. However, cells defective in the pyr4 gene are resistant to this inhibitor but require uridine for growth. It is, therefore, possible to select for pyr4 derivative strains using FOA. In practice, spores of T. reesei strain RL-P37 (Sheir-Neiss, G. and Montenecourt, B.S., Appl. Microbiol. Biotechnol. 20, p. 46-53 (1984)) were spread on the surface of a solidified medium containing 2 mg/ml uridine and 1.2 mg/ml FOA. Spontaneous FOA-resistant colonies appeared within three to four days and it was possible to subsequently identify those FOA-resistant derivatives which required uridine for growth. In order to identify those derivatives which specifically had a defective pyr4 gene, protoplasts were generated and transformed with a plasmid containing a wild-type pyr4 gene (see Examples 3 and 4). Following transformation, protoplasts were plated on medium lacking uridine. Subsequent growth of transformed colonies demonstrated complementation of a defective pyr4 gene by the plasmid-borne pyr4 gene. In this way, strain GC69 was identified as a pyr4 derivative of strain RL-P37.

Example 2

Preparation of CBHI Deletion Vector

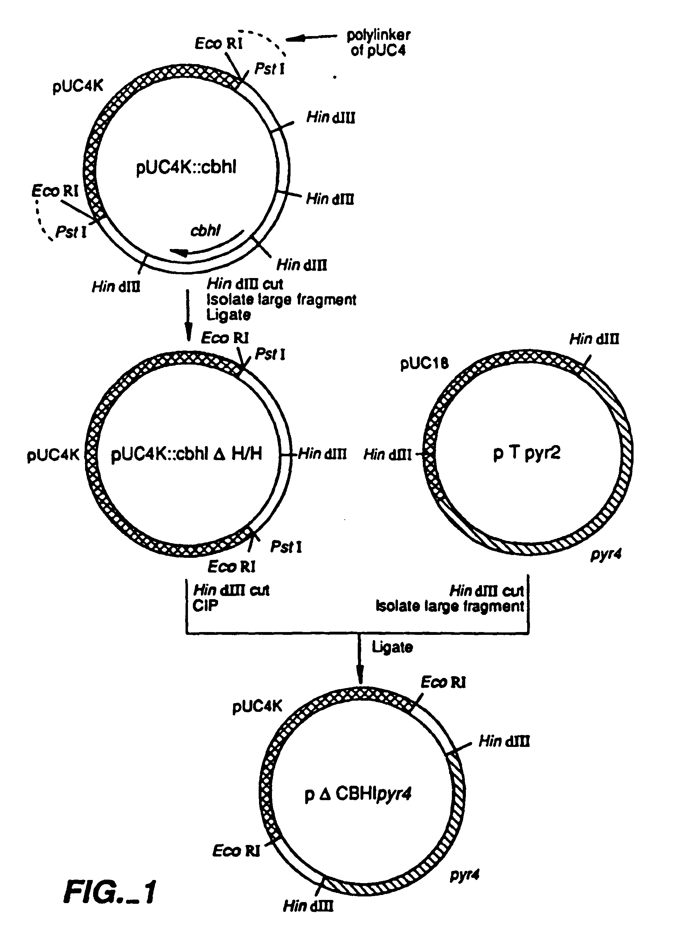

A cbh1 gene encoding the CBHI protein was cloned from the genomic DNA of T. reesei strain RL-P37 by hybridization with an oligonucleotide probe designed on the basis of the published sequence for this gene using known probe synthesis methods (Shoemaker et al., 1983b). The cbh1 gene resides on a 6.5 kb PstI fragment and was inserted into PstI cut pUC4K (purchased from Pharmacia Inc., Piscataway, NJ) replacing the Kan' gene of this vector using techniques known in the art, which techniques are set forth in Maniatis et al., (1989) and incorporated herein by reference. The resulting plasmid, pUC4K::cbh1 was then cut with HindIII and the larger fragment of about 6 kb was isolated and religated to give pUC4K::cbh1ΔH/H (see FIG. 1). This procedure removes the entire cbh1 coding sequence and approximately 1.2 kb upstream and 1.5 kb downstream of flanking sequences. Approximately, 1 kb of flanking DNA from either end of the original PstI fragment remains.

The T. reesei pyr4 gene was cloned as a 6.5 kb HindIII fragment of genomic DNA in pUC18 to form pTpyr2 (Smith et al., 1991) following the methods of Maniatis et al., supra. The plasmid pUC4K::cbhlΔH/H was cut with HindIII and the ends were dephosphorylated with calf intestinal alkaline phosphatase. This end dephosphorylated DNA was ligated with the 6.5 kb HindIII fragment containing the T. reesei pyr4 gene to give pΔCBHIpyr4. FIG. 1 illustrates the construction of this plasmid.

## Example 3

### Isolation of Protoplasts

Mycelium was obtained by inoculating 100 ml of YEG (0.5% yeast extract, 2% glucose) in a 500 ml flask with about $5 \times 10^7$ T. reesei GC69 spores (the pyr4- derivative strain). The flask was then incubated at 37°C with shaking for about 16 hours. The mycelium was harvested by centrifugation at 2,750 x g. The harvested mycelium was further washed in a 1.2 M sorbitol solution and resuspended in 40 ml of a solution containing 5 mg/ml Novozym$^R$ 234 solution (which is the tradename for a multicomponent enzyme system containing 1,3-alpha-glucanase, 1,3-beta-glucanase, laminarinase, xylanase, chitinase and protease from Novo Biolabs, Danbury, Ct.);

5 mg/ml $MgSO_4.7H_2O$; 0.5 mg/ml bovine serum albumin; 1.2 M sorbitol. The protoplasts were removed from the cellular debris by filtration through Miracloth (Calbiochem Corp, La Jolla, CA) and collected by centrifugation at 2,000 x g. The protoplasts were washed three times in 1.2 M sorbitol and once in 1.2 M sorbitol, 50 mM $CaCl_2$, centrifuged and resuspended at a density of approximately $2 \times 10^8$ protoplasts per ml of 1.2 M sorbitol, 50 mM $CaCl_2$.

## Example 4

### Transformation of Fungal Protoplasts with pΔCBHlpyr4

200 μl of the protoplast suspension prepared in Example 3 was added to 20 μl of EcoRI digested pΔCBHlpyr4 (prepared in Example 2) in TE buffer (10 mM Tris, pH 7.4; 1 mM EDTA) and 50 μl of a polyethylene glycol (PEG) solution containing 25% PEG 4000, 0.6 M KCI and 50 mM $CaCl_2$. This mixture was incubated on ice for 20 minutes. After this incubation period 2.0 ml of the above-identified PEG solution was added thereto, the solution was further mixed and incubated at room temperature for 5 minutes. After this second incubation, 4.0 ml of a solution containing 1.2 M sorbitol and 50 mM $CaCl_2$ was added thereto and this solution was further mixed. The protoplast solution was then immediately added to molten aliquots of Vogel's Medium N (3 grams sodium citrate, 5 grams $KH_2PO_4$, 2 grams $NH_4NO_3$, 0.2 grams $MgSO_4.7H_2O$, 0.1 gram $CaCl_2.2H_2O$, 5 μg α-biotin, 5 mg citric acid, 5 mg $ZnSO_4.7H_2O$, 1 mg $Fe(NH_4)_2.6H_2O$, 0.25 mg $CuSO_4.5H_2O$, 50 μg MnSO4.4H20 per liter) containing an additional 1% glucose, 1.2 M sorbitol and 1% agarose. The protoplast/medium mixture was then poured onto a solid medium containing the same Vogel's medium as stated above. No uridine was present in the medium and therefore only transformed colonies were able to grow as a result of complementation of the pyr4 mutation of strain GC69 by the wild type pyr4 gene insert in pΔCBHlpyr4. These colonies were subsequently transferred and purified on a solid Vogel's medium N containing as an additive, 1% glucose and stable transformants were chosen for further analysis.

At this stage stable transformants were distinquished from unstable transformants by their faster growth rate and formation of circular colonies with a smooth, rather than ragged outline on solid culture medium lacking uridine. In some cases a further test of stability was made by growing the transformants on solid non-selective medium (i.e. containing uridine), harvesting spores from this medium and determining the percentage of these spores which will subsequently germinate and grow on selective medium lacking uridine.

## Example 5

### Analysis of the Transformants

DNA was isolated from the transformants obtained in Example 4 after they were grown in liquid Vogel's medium N containing 1 % glucose. These transformant DNA samples were further cut with a PstI restriction enzyme and subjected to agarose gel electrophoresis. The gel was then blotted onto a Nytran membrane filter and hybridized with a $^{32}$P labelled pΔCBHlpyr4 probe. The probe was selected to identify the native cbh1 gene as a 6.5 kb PstI fragment, the native pyr4 gene and any DNA sequences derived from the transforming DNA fragment.

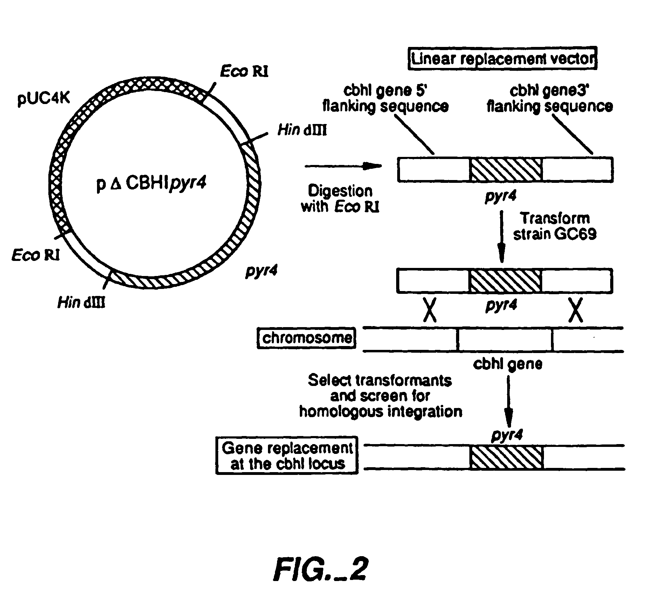

The radioactive bands from the hybridization were visualized by autoradiography. The autoradiograph is seen in FIG. 3. Five samples were run as described above, hence samples A, B, C, D, and E. Lane E is the untransformed strain GC69 and was used as a control in the present analysis. Lanes A-D represent transformants obtained by the methods described above. The numbers on the side of the autoradiograph represent the sizes of molecular weight markers. As can be seen from this autoradiograph, lane D does not contain the 6.5 kb CBHI band, indicating that this gene has been totally deleted in the transformant by integration of the DNA fragment at the cbh1 gene. The cbh1 deleted strain is called P37PΔCBHI. Figure 2 outlines the deletion of the T. reesei cbh1 gene by integration through a double cross-over event of the larger EcoRI fragment from pΔCBHlpyr4 at the cbh1 locus on one of the T. reesei chromosomes. The other transformants analyzed appear identical to the untransformed control strain.

## Example 6

### Analysis of the Transformants with pIntCBHI

The same procedure was used in this example as in Example 5, except that the probe used was changed to a $^{32}$P labelled plntCBHI probe. This probe is a pUC-type plasmid containing a 2 kb BgIII fragment from the cbh1 locus within the region that was deleted in pUC4K::cbh1ΔH/H. Two samples were run in this example including a control, sample A, which is the untransformed strain GC69 and the transformant P37PΔCBHI, sample B. As can be seen in FIG. 4, sample A contained the cbh1 gene, as indicated by the band at 6.5 kb; however the transformant, sample B, does not contain this 6.5 kb band and therefore does not contain the cbh1 gene and does not contain any sequences derived from the pUC plasmid.

## Example 7

### Protein Secretion by Strain P37PΔCBHI

Spores from the produced P37PΔCBHI strain were inoculated into 50 ml of a Trichoderma basal medium containing 1% glucose, 0.14% $(NH_4)_2SO_4$, 0.2% $KH_2PO_4$, 0.03% $MgSO_4$, 0.03% urea, 0.75% bactotryptone, 0.05% Tween 80, 0.000016% $CuSO_4.5H_2O$, 0.001% $FeSO_4.7H_2O$, 0.000128% $ZnSO_4.7H_2O$, 0.0000054% $Na_2MoO_4.2H_2O$, 0.0000007% $MnCl.4H2O$). The medium was incubated with shaking in a 250 ml flask at 37°C for about 48 hours. The resulting mycelium was collected by filtering through Miracloth (Calbiochem Corp.) and washed two or three times with 17 mM potassium phosphate. The mycelium was finally suspended in 17 mM potassium phosphate with 1 mM sophorose and further incubated for 24 hours at 30°C with shaking. The supernatant was then collected from these cultures and the mycelium was discarded. Samples of the culture supernatant were analyzed by isoelectric focusing using a Pharmacia Phastgel system and pH 3-9 precast gels according to the manufacturer's instructions. The gel was stained with silver stain to visualize the protein bands. The band corresponding to the cbh1 protein was absent from the sample derived from the strain P37PΔCBHI, as shown in FIG. 5. This isoelectric focusing gel shows various proteins in different supernatant cultures of T. reesei. Lane A is partially purified CBHI; Lane B is the supernatant from an untransformed T. reesei culture; Lane C is the supernatant from strain P37PΔCBHI produced according to the methods of the present invention. The position of various cellulase components are labelled CBHI, CBHII, EGI, EGII, and EGIII. Since CBHI constitutes 50% of the total extracellular protein, it is the major secreted protein and hence is the darkest band on the gel. This isoelectric focusing gel clearly shows depletion of the CBHI protein in the P37PΔCBHI strain.

## Example 8

### Preparation of pPΔCBHII

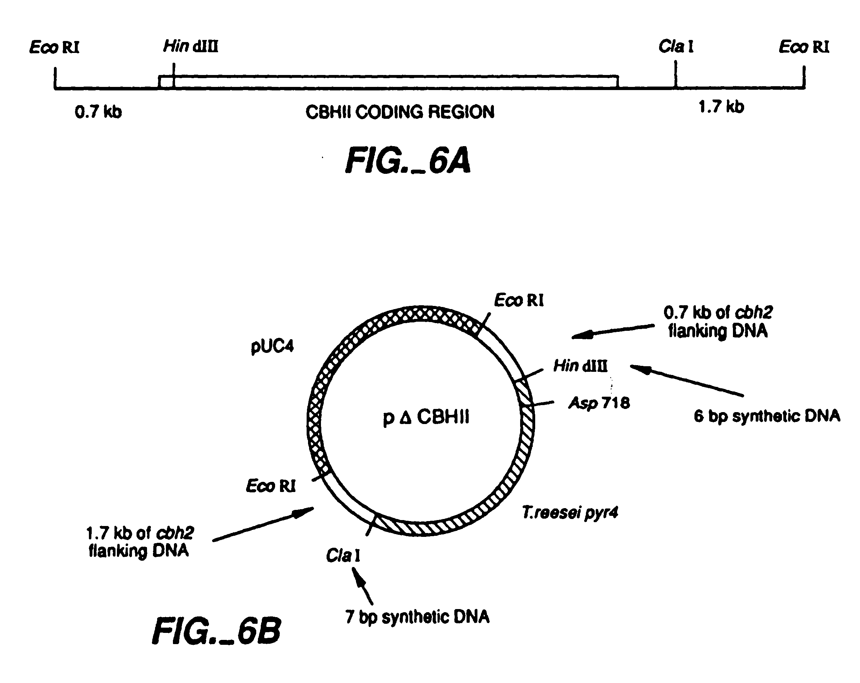

The cbh2 gene of T. reesei, encoding the CBHII protein, has been cloned as a 4.1 kb EcoRI fragment of (Chen et al., 1987, Biotechnology. 5:274-278). This 4.1 kb fragment was inserted between the EcoRI sites of pUC4XL. The latter plasmid is a pUC derivative (constructed by R.M. Berka, Genencor International Inc.) which contains a multiple cloning site with a symetrical pattern of restriction endonuclease sites arranged in the order shown here: EcoRI, BamHI, SacI, SmaI, HindIII, XhoI, BgIII, ClaI, BgIII, XhoI, HindIII, SmaI, SacI, BamHI, EcoRI. Using methods known in the art, a plasmid, pPΔCBHII (FIG. 6B), has been constructed in which a 1.7 kb central region of this gene between a HindIII site (at 74 bp 3' of the CBHII translation initiation site) and a ClaI site (at 265 bp 3' of the last codon of CBHII) has been removed and replaced by a 1.6 kb HindIII- ClaI DNA fragment containing the T. reesei pyr4 gene.

The T. reesei pyr4 gene was excised from pTpyr2 (see Example 2) on a 1.6 kb NheI-SphI fragment and inserted between the SphI and XbaI sites of pUC219 (see Example 23) to create p219M (Smith et al., 1991, Curr. Genet 19 p. 27-33). The pyr4 gene was then removed as a HindIII-ClaI fragment having seven bp of DNA at one end and six bp of DNA at the other end derived from the pUC219 multiple cloning site and inserted into the HindIII and ClaI sites of the cbh2 gene to form the plasmid pPΔCBHII (see FIG. 6B).

Digestion of this plasmid with EcoRI will liberate a fragment having 0.7 kb of flanking DNA from the cbh2 locus at one end, 1.7 kb of flanking DNA from the cbh2 locus at the other end and the T. reesei pyr4 gene in the middle.

Example 9

Deletion of the cbh2 gene in T. reesei strain GC69

Protoplasts of strain GC69 will be generated and transformed with EcoRI digested pPΔCBHI according to the methods outlined in Examples 3 and 4. DNA from the transformants will be digested with EcoRI and Asp718, and subjected to agarose gel electrophoresis. The DNA from the gel will be blotted to a membrane filter and hybridized with 32P labelled pPΔCBHII according to the methods in Example 11. Transformants will be identified which have a single copy of the EcoRI fragment from pPΔCBHII integrated precisely at the cbh2 locus. The transformants will also be grown in shaker flasks as in Example 7 and the protein in the culture supernatants examined by isoelectric focusing. In this manner T. T. reesei GC69 transformants which do not produce the CBHII protein will be generated.

Example 10

Generation of a pyr4- Derivative of P37PΔCBHI

Spores of the transformant (P37PΔCBHI) which was deleted for the cbh1 gene were spread onto medium containing FOA. A pyr4- derivative of this transformant was subsequently obtained using the methods of Example 1. This pyr4- strain was designated P37PΔCBHIPyr⁻26.

Example 11

Deletion of the cbh2 gene in a strain previously deleted for cbh1

Protoplasts of strain P37PΔCBHIPyr⁻26 were generated and transformed with EcoRI digested pPΔCBHII according to the methods outlined in Examples 3 and 4.

Purified stable transformants were cultured in shaker flasks as in Example 7 and the protein in the culture supernatants was examined by isoelectric focusing. One transformant (designated P37PΔΔCBH67) was identified which did not produce any CBHII protein. Lane D of FIG. 5 shows the supernatant from a transformant deleted for both the cbh1 and cbh2 genes produced according to the methods of the present invention.

DNA was extracted from strain P37PΔΔCBH67, digested with EcoRI and Asp718, and subjected to agarose gel electrophoresis. The DNA from this gel was blotted to a membrane filter and hybridized with 32P labelled pPΔCBH II (FIG. 7). Lane A of FIG. 7 shows the hybridization pattern observed for DNA from an untransformed T. reesei strain. The 4.1 kb EcoRI fragment containing the wild-type cbh2 gene was observed. Lane B shows the hybridization pattern observed for strain P37PΔΔCBH67. The single 4.1 kb band has been eliminated and replaced by two bands of approximately 0.9 and 3.1 kb. This is the expected pattern if a single copy of the EcoRI fragment from pPΔCBHII had integrated precisely at the cbh2 locus.

The same DNA samples were also digested with EcoRI and Southern blot analysis was performed as above. In this Example, the probe was 32P labelled pIntCBHII. This plasmid contains a portion of the cbh2 gene coding sequence from within that segment of the cbh2 gene which was deleted in plasmid pPΔCBHII. No hybridization was seen with DNA from strain P37PΔΔCBH67 showing that the cbh2 gene was deleted and that no sequences derived from the pUC plasmid were present in this strain.

Example 12

Construction of pEGIpyr4

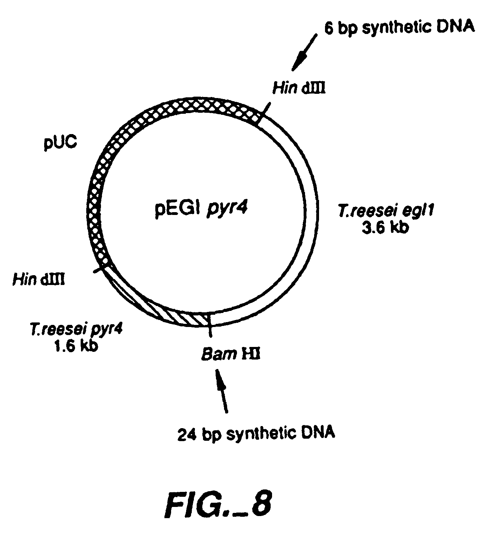

The T. reessi egl1 gene, which encodes EGI, has been cloned as a 4.2 kb HindIII fragment of genomic DNA from strain RL-P37 by hybridization with oligonucleotides synthesized according to the published sequence (Penttila et al., 1986, Gene 45:253-263; van Arsdell et al., 1987, Bio/Technology 5:60-64). A 3.6 kb HindIII-BamHI fragment was taken from this clone and ligated with a 1.6 kb HindIII-BamHI fragment containing the T. reesei pyr4 gene obtained from pTpyr2 (see Example 2) and pUC218 (identical to pUC219, see Example 23, but with the multiple cloning site in the opposite orientation) cut with HindIII to give the plasmid pEGIpyr4 (FIG. 8). Digestion of pEGIpyr4 with HindIII would liberate a fragment of DNA containing only T. reesei genomic DNA (the egl1 and pyr4 genes) except for 24 bp of sequenced, synthetic DNA between the two genes and 6 bp of sequenced, synthetic DNA at one end (see FIG. 8).

Example 13 demonstrates the isolation of the components of Cytolase 123 Cellulase (a complete fungal cellulase composition obtained from Trichoderma reesei and available from Genencor International, Inc., South San Francisco, CA) via purification procedures.

Example 13

Purification of Cytolase 123 Cellulase into Cellulase Components

CYTOLASE 123 cellulase was fractionated in the following manner. The normal distribution of cellulase components in this cellulase system is as follows:

| CBHI | 45-55 weight percent |
|------|----------------------|
| CBH II | 13-15 weight percent |
| EG I | 11-13 weight percent |
| EG II | 8-10 weight percent |
| EG III | 1-4 weight percent |
| BG | 0.5-1 weight percent. |

The fractionation was done using columns containing the following resins: Sephadex G-25 gel filtration resin from Sigma Chemical Company (St. Louis, Mo), QA Trisacryl M anion exchange resin and SP Trisacryl M cation exchange resin from IBF Biotechnics (Savage, Md). CYTOLASE 123 cellulase, 0.5g, was desalted using a column of 3 liters of Sephadex G-25 gel filtration resin with 10 mM sodium phosphate buffer at pH 6.8. The desalted solution, was then loaded onto a column of 20 ml of QA Trisacryl M anion exchange resin. The fraction bound on this column contained CBH I and EG I. These components were separated by gradient elution using an aqueous gradient containing from 0 to about 500 mM sodium chloride. The fraction not bound on this column contained CBH II and EG II. These fractions were desalted using a column of Sephadex G-25 gel filtration resin equilibrated with 10 mM sodium citrate, pH 3.3. This solution, 200 ml, was then loaded onto a column of 20 ml of SP Trisacryl M cation exchange resin. CBH II and EG II were eluted separately using an aqueous gradient containing from 0 to about 200 mM sodium chloride.

Following procedures similar to that of Example 13 above, other cellulase systems which can be separated into their components include CELLUCAST (available from Novo Industry, Copenhagen, Denmark), RAPIDASE (available from Gist Brocades, N.V., Delft, Holland), and cellulase systems derived from Trichoderma koningii, Penicillum sp. and the like.

Example 14

Purification of EG III from Cytolase 123 Cellulase

Example 13 above demonstrated the isolation of several components from Cytolase 123 Cellulase. However, because EG III is present in very small quantities in Cytolase 123 Cellulase, the following procedures were employed to isolate this component.

A. Large Scale Extraction of EG III Cellulase Enzyme

One hundred liters of cell free cellulase filtrate were heated to about 30°C. The heated material was made about 4% wt/vol PEG 8000 (polyethylene glycol, MW of about 8000) and about 10% wt/vol anhydrous sodium sulfate. The mixture formed a two phase liquid mixture. The phases were separated using an SA-1 disk stack centrifuge. The phases were analyzed using silver staining isoelectric focusing gels. Fractionation and enrichment were obtained for EG III and xylanase. The recovered composition contained about 20 to 50 weight percent of EG III.

Regarding the above procedure, use of a polyethylene glycol having a molecular weight substantially less than about 8000 gave inadequate separation; whereas, use of polyethylene glycol having a molecular weight substantially greater than about 8000 resulted in the exclusion of desired enzymes in the recovered composition. With regard to the amount of sodium sulfate, sodium sulfate levels substantially greater than about 10% wt/vol caused precipitation problems; whereas, sodium sulfate levels substantially less than about 10%wt/vol gave poor separation or the solution remained in a single phase.

B. Purification of EG III Via Fractionation

The purification of EG III is conducted by fractionation from a complete fungal cellulase composition (CYTOLASE 123 cellulase, commercially available from Genencor International, South San Francisco, CA) which is produced by wild type Trichoderma reesei. Specifically, the fractionation is done using columns containing the following resins: Sephadex G-25 gel filtration resin from Sigma Chemical Company (St. Louis, Mo), QA Trisacryl M anion exchange resin and SP Trisacryl M cation exchange resin from IBF Biotechnics (Savage, Md). CYTOLASE 123 cellulase, 0.5g, is desalted using a column of 3 liters of Sephadex G-25 gel filtration resin with 10 mM sodium phosphate buffer at pH 6.8. The desalted solution, is then loaded onto a column of 20 ml of QA Trisacryl M anion exchange resin. The fraction bound on this column contained CBH I and EG I. The fraction not bound on this column contains CBH II, EG II and EG III. These fractions are desalted using a column of Sephadex G-25 gel filtration resin equilibrated with 10 mM sodium citrate, pH 4.5. This solution, 200 ml, is then loaded onto a column of 20 ml of SP Trisacryl M cation exchange resin. The EG III was eluted with 100 mL of an aqueous solution of 200 mM sodium chloride.

In order to enhance the efficiency of the isolation of EG III, it may be desirable to employ Trichoderma reesei genetically modified so as to overexpress EG III and/or to be incapable of producing one or more of EG I, EG II, CBH I and/or CBH II components. This will necessarily lead to the more efficient isolation of EG III by, for example, fractionation and/or PEG extraction as described above.

Likewise, it may be desirable for the EG III compositions described above to be further purified to provide for substantially pure EG III compositions, i.e., compositions containing EG III at greater than about 80 weight percent of protein. For example, such a substan-tially pure EG III protein can be obtained by utilizing material obtained from procedure A in procedure B or vica versa. One particular method for further purifying EG III is by further fractionation of an EG III sample obtained in part b) of this Example 14. The further fraction was done on a FPLC system using a Mono-S-HR 5/5 column (available from Pharmacia LKB Biotechnology, Piscataway, NJ). The FPLC system consists of a liquid chromatography controller, 2 pumps, a dual path monitor, a fraction collector and a chart recorder (all of which are available from Pharmacia LKB Biotechnology, Piscataway, NJ). The fractionation was conducted by desalting 5 ml of the EG III sample prepared in part b) of this Example 14 with a 20 ml Sephadex G-25 column which had been previously equilibrated with 10 mM sodium citrate pH 4. The column was then eluted with 0-200 mM aqueous gradient of NaCl at a rate of 0.5 ml/minute with samples collected in 1 ml fractions. EG III was recovered in fractions 10 and 11 and was determined to be greater than 90% pure by SDS gel electrophoresis. EG III of this purity is suitable for determining the N-terminal amino acid sequence by known techniques.

Substantially pure EG III as well as EG I and EG II components purified in Example 13 above can be used singularly or in mixtures in the methods of this invention. These EG components have the following characteristics:

|  | MW | pl | pH optimum [1] |
|---|---|---|---|
| EG I | -47-49 kD | 4.7 | -5 |
| EG II | -35 kD | 5.5 | -5 |
| EG III | -25-28 kD | 7.4 | -5.5-6.0 |
| 1. pH optimum determined by RBB-CMC activity as per Example 15 below. | | | |

The use of a mixture of these components in the practice of this invention may give a synergistic response in improving softening, color retention/restoration and/or feel as compared to a single component. On the other hand, the use of a single component in the practice of this invention may be more stable or have a broader spectrum of activity over a range of pHs. For instance, Example 15 below shows that EG III has considerable activity against RBB-CMC under alkaline conditions.

Example 15

Activity of Cellulase Compositions Over a pH Range

The following procedure was employed to determine the pH profiles of two different cellulase compositions. The first cellulase composition was a CBH I and II deleted cellulase composition prepared from Trichoderma reesei genetically modified in a manner similar to that described above so as to be unable to produce CBH I and CBH II components. Insofar as this cellulase composition does not contain CBH I and CBH II which generally comprise from about 58 to 70 percent of a cellulase composition derived from Trichoderma reesei, this cellulase composition is necessarily enriched in EG components, i.e., EG I, EG II, EG III and the like.

The second cellulase composition was an approximately 20-40% pure fraction of EG III isolated from a cellulase composition derived from Trichoderma reesei via purification methods similar to part b) of Example 14.

The activity of these cellulase compositions was determined at 40°C and the determinations were made using the following procedures.

Add 5 to 20 µl of an appropriate enzyme solution at a concentration sufficient to provide the requisite amount of enzyme in the final solution. Add 250 µl of 2 weight percent RBB-CMC (Remazol Brilliant Blue R-Carboxymethyl-cellulose - commercially available from MegaZyme, 6 Altona Place, North Rocks, N.S.W. 2151, Australia) in 0.05M citrate/phosphate buffer at pH 4, 5, 5.5, 6, 6.5, 7, 7.5 and 8.

Vortex and incubate at 40°C for 30 minutes. Chill in an ice bath for 5 to 10 minutes. Add 1000 pl of methyl cellosolve containing 0.3M sodium acetate and 0.02M zinc acetate. Vortex and let sit for 5-10 minutes. Centrifuge and pour supernatant into cuvets. Relative enzyme activity was determined by measuring the optical density (OD) of the solution in each cuvet at 590 nm. Higher levels of optical density correspond to higher levels of enzyme activity.

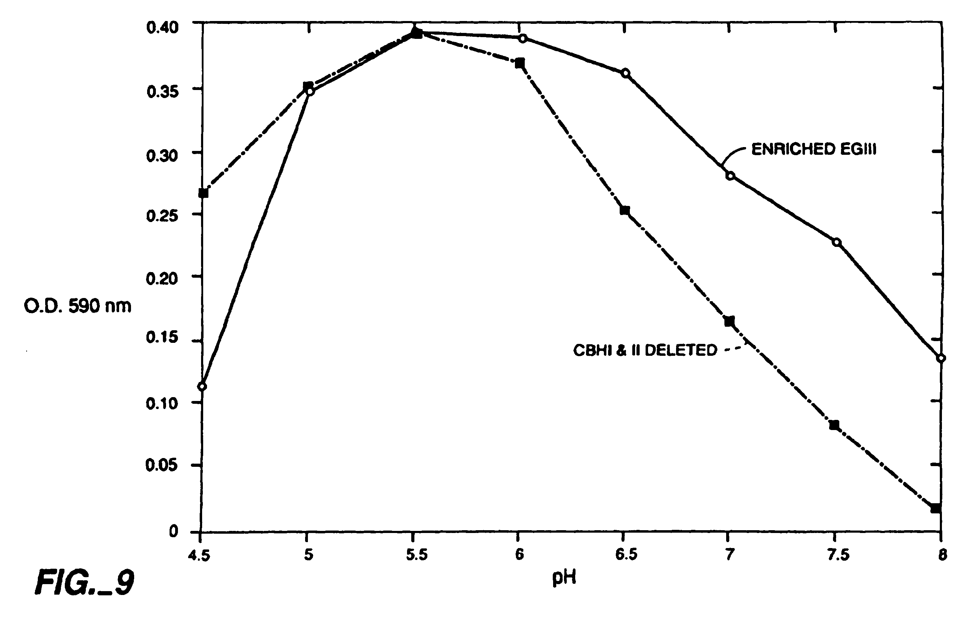

The results of this analysis are set forth in FIG. 9 which illustrates the relative activity of the CBH I and II deleted cellulase composition compared to the EG III cellulase composition. From this figure, it is seen that the cellulase composition deleted in CBH I and CBH II possesses optimum cellulolytic activity against RBB-CMC at near pH 5.5 and has some activity at alkaline pHs, i.e., at pHs from above 7 to 8. On the other hand, the cellulase composition enriched in EG III possesses optimum cellulolytic activity at about pH 5.5 - 6 and possesses significant activity at alkaline pHs.

Example 16

Launderometer Strength Loss Assay Cellulase Compositions

The purpose of this example is to examine the ability of different cellulase compositions to reduce the strength of cotton-containing fabrics. Because the activity of most of the cellulase components derived from Trichoderma reesei is greatest at or near pH 5, strength loss results will be most evident when the assay is conducted at about this pH. However, the results achieved at pH 5 are believed to correlate with strength loss results which would occur at other pHs and accordingly, are indicative of the relative strength loss capacity of the cellulase components analyzed.

Specifically, in this example, the first cellulase composition analyzed was a complete fungal cellulase composition (CYTOLASE 123 cellulase, commercially available from Genencor International, South San Francisco, CA) produced by wild type Trichoderma reesei and is identified as GC010.

The second cellulase composition analyzed was a CBH II deleted cellulase composition prepared from Trichoderma reesei genetically modified in a manner similar to Examples 1-12 above and 20-28 below so as to be incapable of expressing CBH II and is identified as CBHIId. Insofar as CBH II comprises up to about 15 percent of the cellulase composition, deletion of this component results in enriched levels of CBH I, and all of the EG components.

The third cellulase composition analyzed was a CBH I and CBH II deleted cellulase composition prepared from Trichoderma reesei genetically modified in a manner similar to that described above so as to be incapable of expressing CBH I and CBH II and is identified as CBHI/IId. Insofar as CBH I and CBH 11 comprises up to about 70 percent of the cellulase composition, deletion of this component results in enriched levels of all of the EG components.

The last cellulase composition analyzed was a CBH I deleted cellulase composition prepared from Trichoderma reesei genetically modified in a manner similar to that described above so as to be incapable of expressing CBH I and is identified as CBHId. Insofar as CBH I comprises up to about 55 percent of the cellulase composition, deletion of this component results in enriched levels of CBH II and all of the EG components.

The cellulase compositions described above were tested for their effect on cotton-containing fabric strength loss in a launderometer. The compositions were first normalized so that equal amounts of EG components were used. Each cellulase composition was then added to separate solutions of 400 ml of a 20 mM citrate/phosphate buffer, titrated to pH 5, and which contains 0.5 ml of a non-ionic surfactant. Each of the resulting solutions was then added to a separate launderometer canister. Into these canisters were added a quantity of marbles to facilitate strength loss as well as a 16 inch x 20 inch cotton fabric (100% woven cotton, available as Style No. 467 from Test Fabrics, Inc., 200 Blackford Ave., Middlesex, NJ 08846). The canister was then closed and the canister lowered into the launderometer bath which was maintained at 43°C. The canister was then rotated in the bath at a speed of at least about 40 revolutions per minute (rpms) for about 1 hour. Afterwards, the cloth is removed, rinsed well and dried in a standard drier.

In order to maximize strength loss results, the above procedure was repeated twice more and after the third treatment, the cotton fabrics were removed and analyzed for strength loss. Strength loss was measured by determining the tensile strength in the fill direction ("FTS") using a Instron Tester and the results compared to the FTS of the fabric treated in the same solution with the exception that no cellulase was added. The results of this analysis are reported as % strength loss which is determined as follows:

$$\% \text{ Strength Loss} = 100 \times \left[ 1 - \frac{\text{FTS with cellulase}}{\text{FTS without cellulase}} \right]$$

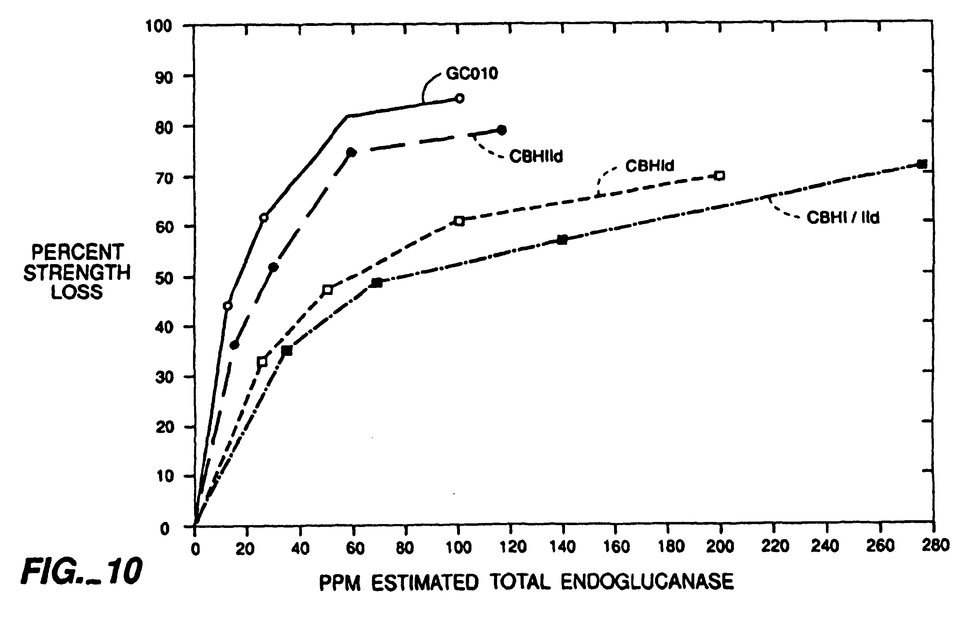

The results of this analysis are set forth in FIG. 10 which shows that compositions containing CBH I, i.e., whole cellulase (GC010) and CBH II deleted cellulase, possessed the most strength loss whereas, the compositions containing no CBH I possessed significantly reduced strength loss as compared to whole cellulase and CBH II deleted cellulase. From these results, it is seen that the presence of CBH I in a cellulase composition imparts increased strength loss to the composition as compared to a similar composition not containing CBH I.

Likewise, these results show that CBH II plays some role in strength loss.

Accordingly, in view of these results, when strength loss resistant cellulase compositions are desired, such compositions should be free of all CBH I type cellulase components and preferably, all CBH type cellulase components. In this regard, it is contemplated that EG enriched cellulase compositions will result in even lower strength loss at pH. >_ 7 than those results observed at pH 5 shown in FIG. 10.

Example 17

Color Restoration

The ability of EG enriched cellulase to restore color in cotton-containing fabrics was analyzed in the following experiments. Specifically, color restoration of a worn cotton fabric arises from the accumulation on the fabric of surface fibers over a period of time. These fibers give rise to a faded and matted appearance for the fabric and accordingly, the removal of these fibers is a necessary prerequisite to restoring the original sharp color to the fabric. In view of the above, these experiments determine the ability of a cellulase composition to restore color by measuring the ability of the cellulase composition to remove surface fibers.

The first experiment measures the ability of a complete cellulase system (CYTOLASE 123 cellulase, commercially available from Genencor International, South San Francisco, CA) produced by wild type Trichoderma reesei to remove surface fibers from a cotton-containing fabric over various pHs. This cellulase was tested for its ability to remove surface fibers in a launderometer. An appropriate amount of cellulase to provide for either 25 ppm or 100 ppm cellulase in the final composition was added to separate solutions of 400 ml of a 20 mM citrate/phos-phate buffer containing 0.5 ml of a non-ionic surfactant. Samples were prepared and titrated so as to provide for samples at pH 5, pH 6, pH 7 and pH 7.5. Each of the resulting solution was then added to a separate launderometer canister. Into these canisters were added a quantity of marbles to facilitate fiber removal as well as a 7 inch x 5 inch cotton fabric (100% woven cotton, available as Style No. 439W from Test Fabrics, Inc., 200 Blackford Ave., Middlesex, NJ 08846). The canister was then closed and the canister lowered into the launderometer bath which was maintained at 43°C. The canister was then rotated in the bath at a speed of at least about 40 revolutions per minute (rpms) for about 1 hour. Afterwards, the cloth is removed, rinsed well and dried in a standard drier.

The so treated fabrics were then analyzed for fiber removal by evaluation in a panel test. In particular, the coded fabrics (i.e., unmarked) were rated for levels of fiber by 6 individuals. The fabrics were visually evaluated for surface fibers and rated on a 0 to 6 scale. The scale has six standards to allow meaningful comparisons. The standards are as follows:

| Rating | Standard[2] |
|--------|-------------|
| 0 | Fabric not treated with cellulase |
| 1 | Fabric treated[3] with 8 ppm cellulase |
| 2 | Fabric treated with 16 ppm cellulase |
| 3 | Fabric treated with 20 ppm cellulase |
| 4 | Fabric treated with 40 ppm cellulase |
| 5 | Fabric treated with 50 ppm cellulase |

(continued)

| Rating | Standard[2] |
|--------|-------------|
| 6 | Fabric treated with 100 ppm cellulase |

| 2 In all of the standards, the fabric was a 100% cotton sheeting standardized test fabric (Style No. 439W) available from Test Fabrics, Inc., 200 Blackford Ave., Middlesex, NJ 08846<br>3 For all samples treated with the same cellulase composition. Cellulase concentrations are in total protein. The launderometer treatment conditions are the same as set forth in Example 16 above. |
|---|

The fabric to be rated was provided a rating which most closely matched one of the standards. After complete analysis of the fabrics, the values assigned to each fabric by all of the individuals were added and an average value generated.

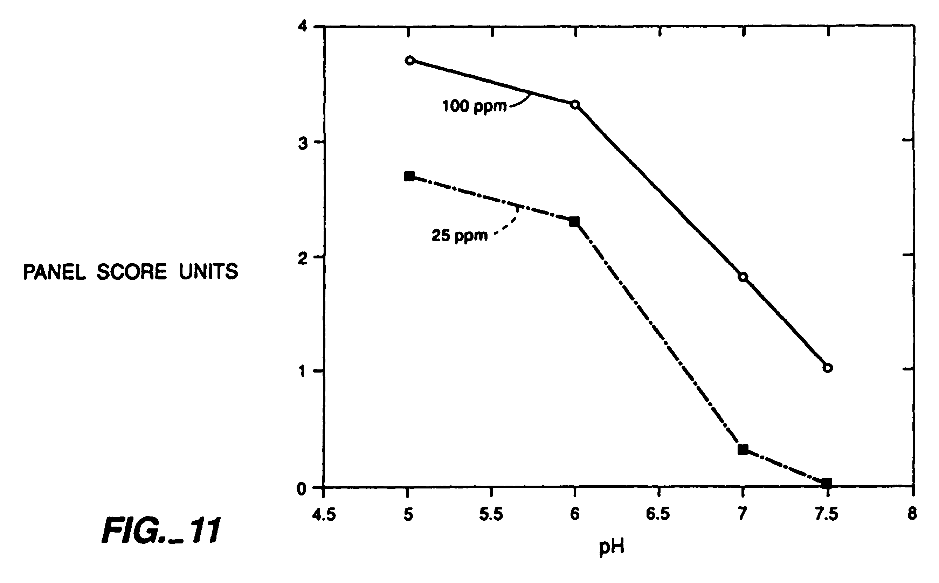

The results of this analysis are set forth in FIG. 11. Specifically, FIG. 11 illustrates that at the same pH, a dose dependent response is seen in the amount of fibers removed. That is to say that at the same pH, the fabrics treated with more cellulase provided for higher levels of fiber removal as compared to fabrics treated with less cellulase. Moreover, the results of this figure demonstrate that at higher pHs, fiber removal can still be effected merely by using higher concentrations of cellulase.

In a second experiment, two different cellulase compositions were compared for the ability to remove fiber. Specifically, the first cellulase composition analyzed was a complete cellulase system (CYTOLASE 123 cellulase, commercially available from Genencor International, South San Francisco, CA) produced by wild type Trichoderma reesei and is identified as GC010.

The second cellulase composition analyzed was a CBH I and CBH II deleted cellulase composition prepared from Trichoderma reesei genetically modified in a manner similar to that described above so as to be incapable of producing CBH I and CBH II and is identified as CBHI/IId. Insofar as CBH I and CBH II comprises up to about 70 percent of the cellulase composition, deletion of this component results in enriched levels of all of the EG components.

The cellulase compositions were first normalized so that similar amounts of EG components were used. These compositions were tested for their ability to remove surface fibers in a launderometer. An appropriate amount of cellulase to provide for the requisite concentrations of EG components in the final compositions were added to separate solutions of 400 ml of a 20 mM citrate/phosphate buffer containing 0.5 ml of a non-ionic surfactant. Samples were prepared and titrated to pH 5. Each of the resulting solutions was then added to a separate launder-ometer canister. Into these canisters were added a quantity of marbles to facilitate fiber removal as well as a 7 inch x 5 inch cotton fabric (100% woven cotton, available as Style No. 439W from Test Fabrics, Inc., 200 Blackford Ave., Middlesex, NJ 08846). The canister was then closed and the canister lowered into the launderometer bath which was maintained at 43°C. The canister was then rotated in the bath at a speed of at least about 40 revolutions per minute (rpms) for about 1 hour. Afterwards, the cloth is removed, rinsed well and dried in a standard drier.

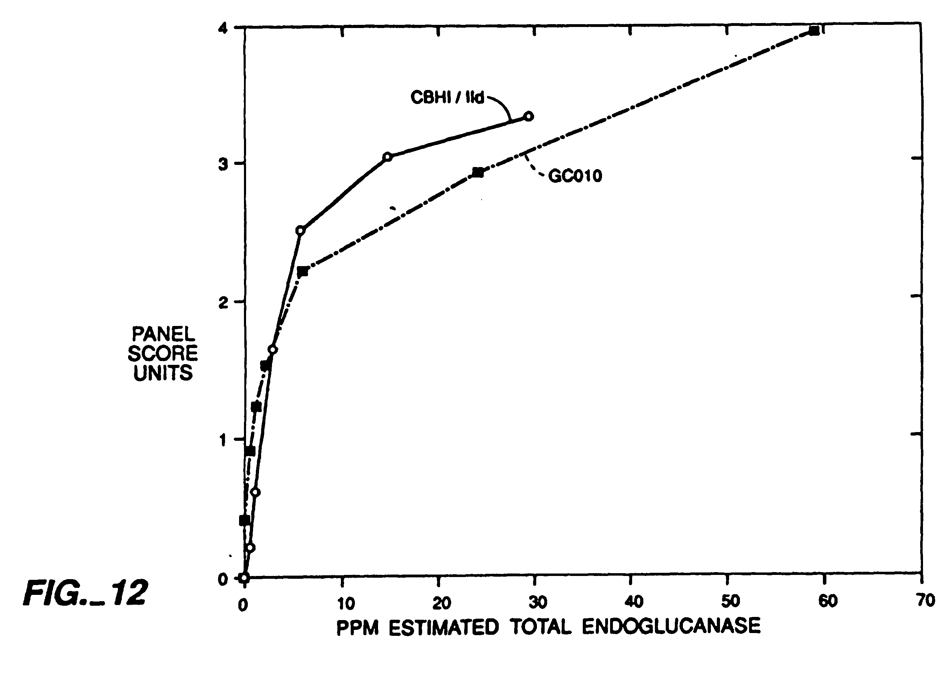

The so treated fabrics were then analyzed for fiber removal by evaluation in the panel test described above. The results of this analysis are set forth in FIG. 12. Specifically, FIG. 12 illustrates that both GC010 and CBH I/IId cellulase compositions gave substantially identical fiber removal results when equal amounts of EG are used. These results suggest that it is the EG components which provide for fiber removal. These results coupled with the results of FIG. 11 demonstrate that EG components can remove fibers even under alkaline conditions.

<u>Example 18</u>

<u>Tergotometer Color Restoration</u>

This example is further to Example 17 and substantiates that CBH type components are not necessary for color restoration. The purpose of this example is to examine the ability of cellulase compositions deficient in CBH type components to restore color to cotton-containing fabrics. Because the activity of most of the EG components derived from Trichoderma reesei is greatest at or near pH 5, color restoration results will be most evident when the assay is conducted at about this pH. However, the results achieved at pH 5 are believed to correlate with color restoration results which would occur at other pHs and accordingly, are indicative of the relative color restoration capacity of the cellulase components analyzed.

Specifically, the cellulase composition employed in this example was a CBH I and CBH II deleted cellulase composition prepared from Trichoderma reesei genetically modified in a manner similar to that described above so as to be incapable of producing CBH I and CBH II. Insofar as CBH I and CBH II comprises up to about 70 percent of the cellulase composition, deletion of this component results in enriched levels of all of the EG components.

The assay was conducted by adding a sufficient concentration of this cellulase composition to a 50 mM citrate/phosphate buffer to provide 500 ppm of cellulase. The solution was titrated to pH 5 and contained 0.1 weight percent of nonionic surfactant (Grescoterg GL100 - commercially available from Gresco Mfg., Thomasville, NC 27360). A 10 inch x 10 inch faded cotton-containing fabric was then placed into 1 liter of this buffer and allowed to sit at 110°F for 30 minutes and then agitated for 30 minutes at 100 rotations per minute. The fabrics were then removed from the buffer, washed and dried. The resulting fabrics were then compared to the fabric prior to treatment. The results of this analysis are as follows:

| Cotton-Containing Material | Result |
| --- | --- |
| Worn Cotton T-Shirt | benefit seen |
| Cotton Knit | benefit seen |

The term "benefit seen" means that the treated fabric exhibits color restoration (i.e., is less faded) as compared to the non-treated fabric. These results substantiate the results of Example 17 that the presence of CBH type components is not necessary for effecting color restoration of faded cotton-containing fabrics.

Example 19

Softness

This example demonstrates that the presence of CBH type components are not essential for imparting improved softness to cotton-containing fabrics. Specifically, this example employs a cellulase composition derived from Trichoderma reesei genetically engineered in the manner described above so as to be incapable of producing CBH I and II components.

This cellulase composition was tested for its ability to soften terry wash cloth. Specifically, unsoftened 8.5 ounce cotton terry cloths, 14 inches by 15 inches (available as Style No. 420NS from Test Fabrics, Inc., 200 Blackford Ave., Middlesex, NJ 08846), were cut into 7 inch by 7.5 inch swatches.

The cellulase composition described above was tested for its ability to soften these swatches in a launderometer. Specifically, an appropriate amount of cellulase to provide for 500 ppm, 250 ppm, 100 ppm, 50 ppm, and 10 ppm cellulase in the final cellulase solution was added to separate solutions of 400 ml of a 20 mM citrate/phosphate buffer containing 0.025 weight percent of a non-ionic surfactant (Triton X114). Additionally, a blank was run containing the same solution but with no added cellulase. Samples so prepared were titrated to pH 5. Each of the resulting solutions was then added to a separate launderometer canister. Into these canisters were added a quantity of marbles to facilitate softness as well as cotton swatches described above. All conditions were run in triplicate with two swatches per canister. Each canister was then closed and the canister lowered into the launderometer bath which was maintained at 37°C. The canister was then rotated in the bath at a speed of at least about 40 revolutions per minute (rpms) for about 1 hour. Afterwards, the swatches were removed, rinsed well and dried in a standard drier.

The swatches were then analyzed for softness by evaluation in a preference test. Specifically, six panelists were given their own set of swatches and ask to rate them with respect to softness based on softness criteria such as the pliability of the whole fabric. Swatches obtained from treatment with the five different enzyme concentrations and the blank were placed behind a screen and the panelists were asked to order them from least soft to most soft. Scores were assigned to each swatch based on its order relative to the other swatches; 5 being most soft and 0 being least soft. The scores from each panelists were cumulated and then averaged.

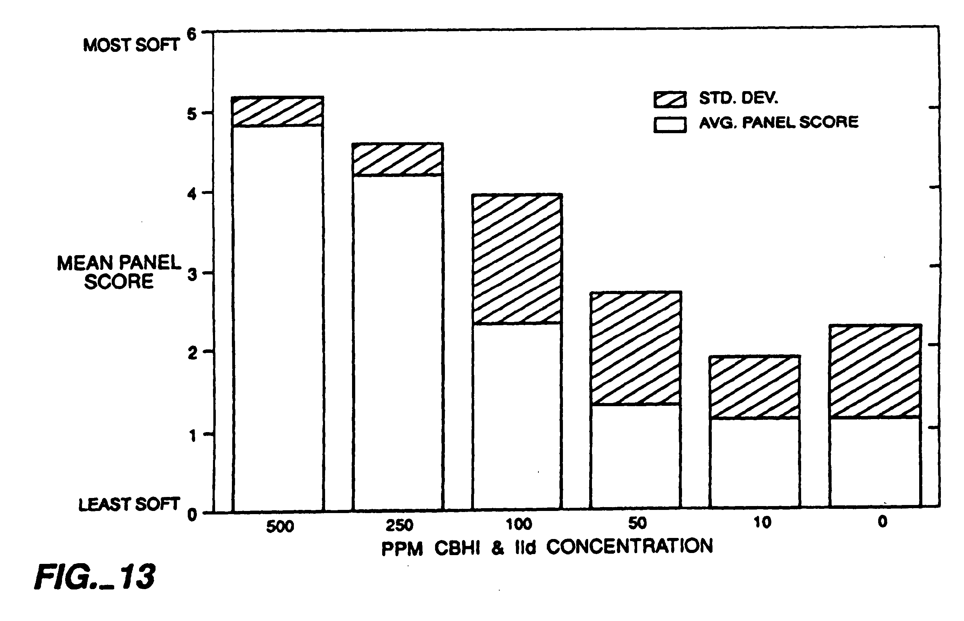

The results of this averaging are set forth in FIG. 13. Specifically, these results demonstrate that at higher cellulase concentrations, improved softening is obtained. It is noted that this improved softening is achieved without the presence of either CBH I or II in the cellulase composition.

With regard to these results, it is contemplated that with lower concentrations of the cellulase composition, improved softening will manifest itself over repeated washings.

With regard to Examples 16 to 19, cellulase compositions enriched in EG type components derived from microorganisms other than Trichoderma reesei could be used in place of the cellulase compositions described in these examples. In particular, the source of the cellulase composition enriched in EG type components is not important to this invention and any fungal cellulase composition enriched in EG type components can be used. For example, fungal cellulases for use in preparing the fungal cellulase compositions used in this invention can be obtained from Trichoderma koningii, Pencillum sp., and the like or commercially available cellulases can be used, i.e., CELLUCLAST (available from Novo Industry, Copenhagen, Denmark), RAPIDASE (available from Gist Brocades, N.V., Delft, Holland), and the like.

Example 20

Transformants of Trichoderma reesei Containing the plasmid DEGIpyr4

A pyr4 defective derivative of T. reesei strain RutC30 (Sheir-Neiss and Montenecourt, (1984), Appl. Microbiol. Biotechnol. 20:46-53) was obtained by the method outlined in Example 1. Protoplasts of this strain were transformed with undigested pEGIpyr4 and stable transformants were purified.

Five of these transformants (designated EP2, EP4, EP5, EP6, EG11), as well as untransformed RutC30 were inoculated into 50 ml of YEG medium (yeast extract, 5 g/l; glucose, 20 g/l) in 250 ml shake flasks and cultured with shaking for two days at 28°C. The resulting mycelium was washed with sterile water and added to 50 ml of TSF medium (0.05M citrate-phosphate buffer, pH 5.0; Avicel microcrystalline cellulose, 10 g/l; $KH_2PO_4$, 2.0 g/l; $(NH_6)_2SO_4$, 1.4 g/l; proteose peptone, 1.0 g/l; Urea, 0.3 g/l; $MgSO_4.7H_2O$, 0.3 g/l; $CaCl_2$, 0.3 g/l; $FeSO_4.7H_2O$, 5.0 mg/l; $MnSO_4.H_2O$, 1.6 mg/l; $ZnSO_4$, 1.4 mg/l; $CoCl_2$, 2.0 mg/l; 0.1% Tween 80). These cultures were incubated with shaking for a further four days at 28°C. Samples of the supernatant were taken from these cultures and assays designed to measure the total amount of protein and of endoglucanase activity were performed as described below.

The endoglucanase assay relied on the release of soluble, dyed oligosaccharides from Remazol Brilliant Blue-carboxymethylcellulose (RBB-CMC, obtained from MegaZyme, North Rocks, NSW, Australia). The substrate was prepared by adding 2 g of dry RBB-CMC to 80 ml of just boiled deionized water with vigorous stirring. When cooled to room temperature, 5 ml of 2 M sodium acetate buffer (pH 4.8) was added and the pH adjusted to 4.5. The volume was finally adjusted to 100 ml with deionized water and sodium azide added to a final concentration of 0.02%. Aliquots of T. reesei control culture, pEGIpyr4 transformant culture supernatant or 0.1 M sodium acetate as a blank (10-20 µl) were placed in tubes, 250 µl of substrate was added and the tubes were incubated for 30 minutes at 37°C. The tubes were placed on ice for 10 minutes and 1 ml of cold precipitant (3.3% sodium acetate, 0.4% zinc acetate, pH 5 with HCl, 76% ethanol) was then added. The tubes were vortexed and allowed to sit for five minutes before centrifuging for three minutes at approximately 13,000 x g. The optical density was measured spectrophotometrically at a wavelength of 590-600 nm.

The protein assay used was the BCA (bicinchoninic acid) assay using reagents obtained from Pierce, Rockford, Illinois, USA. The standard was bovine serum albumin (BSA). BCA reagent was made by mixing 1 part of reagent B with 50 parts of reagent A. One ml of the BCA reagent was mixed with 50 µl of appropriately diluted BSA or test culture supernatant. Incubation was for 30 minutes at 37°C and the optical density was finally measured spectrophotometrically at a wavelength of 562 nm.

The results of the assays described above are shown in Table 1. It is clear that some of the transformants produced increased amounts of endoglucanase activity compared to untransformed strain RutC30. It is thought that the endoglucanases and exo-cellobiohydrolases produced by untransformed T. reesei constitute approximately 20 and 70 percent respectively of the total amount of protein secreted. Therefore a transformant such as EPS, which produces approximately four-fold more endoglucanase than strain RutC30, would be expected to secrete approximately equal amounts of endoglucanase-type and exo-cellobiohydrolase-type proteins.

The transformants described in this Example were obtained using intact pEGIpyr4 and will contain DNA sequences integrated in the genome which were derived from the pUC plasmid. Prior to transformation it would be possible to digest pEGIpyr4 with HindIII and isolate the larger DNA fragment containing only T. reesei DNA. Transformation of T. reesei with this isolated fragment of DNA would allow isolation of transformants which overproduced EGI and contained no heterologous DNA sequences except for the two short pieces of synthetic DNA shown in FIG. 8. It would also be possible to use pEGIpyr4 to transform a strain which was deleted for either the cbh1 gene, or the cbh2 gene, or for both genes. In this way a strain could be constructed which would over-produce EGI and produce either a limited range of, or no, exo-cellobiohydrolases.

The methods of Example 20 could be used to produce T. reesei strains which would over-produce any of the other cellulase components, xylanase components or other proteins normally produced by T. reesei.

TABLE 1

| Secreted Endoglucanase Activity of T. reesei Transformants | | | |
|---|---|---|---|
| | A | B | |
| STRAIN | ENDOGLUCANASE ACTIVITY (O.D. AT 590 nm) | PROTEIN (mg/ml) | A/B |
| RutC30 | 0.32 | 4.1 | 0.078 |
| EP2 | 0.70 | 3.7 | 0.189 |

(continued)

| Secreted Endoglucanase Activity of T. reesei Transformants | | | |
|---|---|---|---|
| | A | B | |
| STRAIN | ENDOGLUCANASE ACTIVITY (O.D. AT 590 nm) | PROTEIN (mg/ml) | A/B |
| EP4 | 0.76 | 3.65 | 0.208 |
| EP5 | 1.24 | 4.1 | 0.302 |
| EP6 | 0.52 | 2.93 | 0.177 |
| EP11 | 0.99 | 4.11 | 0.241 |

The above results are presented for the purpose of demonstrating the overproduction of the EGI component relative to total protein and not for the purpose of demonstrating the extent of overproduction. In this regard, the extent of overproduction is expected to vary with each experiment.

Example 21

Construction of pCEPC1

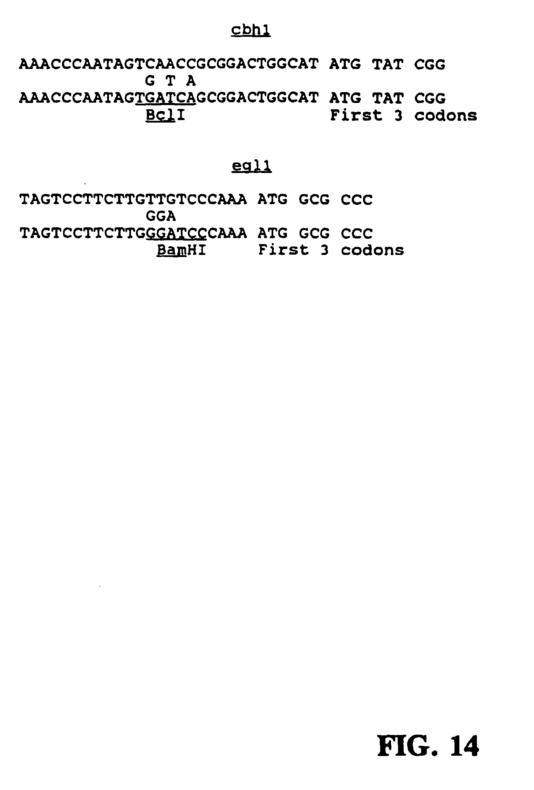

A plasmid, pCEPC1, was constructed in which the coding sequence for EGI was functionally fused to the promoter from the cbh1 gene. This was achieved using in vitro, site-specific mutagenesis to alter the DNA sequence of the cbh1 and egl1 genes in order to create convenient restriction endonuclease cleavage sites just 5' (upstream) of their respective translation initiation sites. DNA sequence analysis was performed to verify the expected sequence at the junction between the two DNA segments. The specific alterations made are shown in FIG. 14.

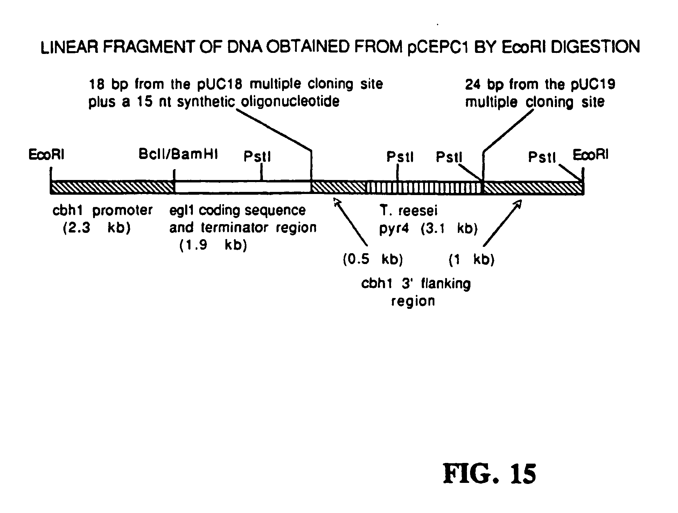

The DNA fragments which were combined to form pCEPC1 were inserted between the EcoRI sites of pUC4K and were as follows (see FIG. 15):

A) A 2.1 kb fragment from the 5' flanking region of the cbh1 locus. This includes the promoter region and extends to the engineered BclI site and so contains no cbh1 coding sequence.

B) A 1.9 kb fragment of genomic DNA from the egl1 locus starting at the 5' end with the engineered BamHI site and extending through the coding region and including approximately 0.5 kb beyond the translation stop codon. At the 3' end of the fragment is 18 bp derived from the pUC218 multiple cloning site and a 15 bp synthetic oligonucleotide used to link this fragment with the fragment below.

C) A fragment of DNA from the 3' flanking region of the cbh1 locus, extending from a position approximately 1 kb downstream to approximately 2.5 kb downstream of the cbh1 translation stop codon.

D) Inserted into an NheI site in fragment (C) was a 3.1 kb NheI-SphI fragment of DNA containing the T. reesei pyr4 gene obtained from pTpyr2 (Example 2) and having 24 bp of DNA at one end derived from the pUC18 multiple cloning site.

The plasmid, pCEPC1 was designed so that the EGI coding sequence would be integrated at the cbh1 locus, replacing the coding sequence for CBHI without introducing any foreign DNA into the host strain. Digestion of this plasmid with EcoRI liberates a fragment which includes the cbh1 promoter region, the egl1 coding sequence and transcription termination region, the T. reesei pyr4 gene and a segment of DNA from the 3' (downstream) flanking region of the cbh1 locus (see Fig. 15).

Example 22

Transformants containing PCEPC1 DNA

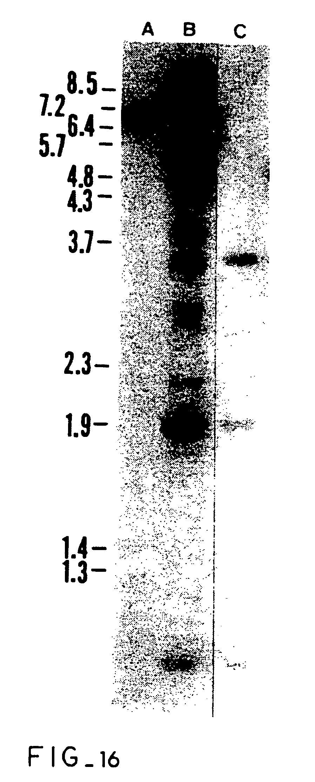

A pyr4 defective strain of T. reesei RutC30 (Sheir-Neiss, supra) was obtained by the method outlined in Example 1. This strain was transformed with pCEPC1 which had been digested with EcoRI. Stable transformants were selected and subsequently cultured in shaker flasks for cellulase production as described in Example 20. In order to visualize the cellulase proteins, isoelectric focusing gel electrophoresis was performed on samples from these cultures using the method described in Example 7. Of a total of 23 transformants analysed in this manner 12 were found to produce no CBHI protein, which is the expected result of integration of the CEPC1 DNA at the cbh1 locus. Southern blot analysis was used to confirm that integration had indeed occurred at the cbh1 locus in some of these transformants and that no sequences derived from the bacterial plasmid vector (pUC4K) were present (see Fig. 16). For this analysis the DNA from the transformants was digested with PstI before being subjected to electrophoresis and blotting to a membrane filter. The resulting Southern blot was probed with radiolabelled plasmid pUC4K::cbh1 (see Example 2). The probe hybridised to the cbh1 gene on a 6.5 kb fragment of DNA from the untransformed control culture (FIG. 16, lane A). Integration of the CEPC1 fragment of DNA at the cbh1 locus would be expected to result in the loss of this 6.5 kb band and the appearance of three other bands corresponding to approximately 1.0 kb, 2.0 kb and 3.5 kb DNA fragments. This is exactly the pattern observed for the transformant shown in FIG. 16, lane C. Also shown in FIG. 16, lane B is an example of a transformant in which multiple copies of pCEPC1 have integrated at sites in the genome other than the cbh1 locus.

Endoglucanase activity assays were performed on samples of culture supernatant from the untransformed culture and the transformants exactly as described in Example 20 except that the samples were diluted 50 fold prior to the assay so that the protein concentration in the samples was between approximately 0.03 and 0.07 mg/ml. The results of assays performed with the untransformed control culture and four different transformants (designated CEPC1-101, CEPC1-103, CEPC1-105 and CEPC1-112) are shown in Table 2. Transformants CEPC1-103 and CEPC1-112 are examples in which integration of the CEPC1 fragment had led to loss of CBHI production.

Table 2

| Secreted endoglucanase activity of T. reesei transformants | | | |
|---|---|---|---|
| | A | B | A/B |
| STRAIN | ENDOGLUCANASE ACTIVITY (O.D. at 590 nm) | PROTEIN (mg/ml) | |
| RutC300 | 0.037 | 2.38 | 0.016 |
| CEPC1-101 | 0.082 | 2.72 | 0.030 |
| CEPC1-103 | 0.099 | 1.93 | 0.051 |
| CEPC1-105 | 0.033 | 2.07 | 0.016 |
| CEPC1-112 | 0.093 | 1.72 | 0.054 |

The above results are presented for the purpose of demonstrating the overproduction of the EGI component relative to total protein and not for the purpose of demonstrating the extent of overproduction. In this regard, the extent of overproduction is expected to vary with each experiment.

It would be possible to construct plasmids similar to pCEPC1 but with any other T. reesei gene replacing the egl1 gene. In this way, overexpression of other genes and simultaneous deletion of the cbh1 gene could be achieved.

It would also be possible to transform pyr4 derivative strains of T. reesei which had previously been deleted for other genes, eg. for cbh2, with pCEPC1 to construct transformants which would, for example, produce no exo-cellobiohydrolases and overexpress endoglucanases.

Using constructions similar to pCEPC1, but in which DNA from another locus of T. reesei was substituted for the DNA from the cbh1 locus, it would be possible to insert genes under the control of another promoter at another locus in the T. reesei genome.

Example 23

Construction of pEGII::P-1

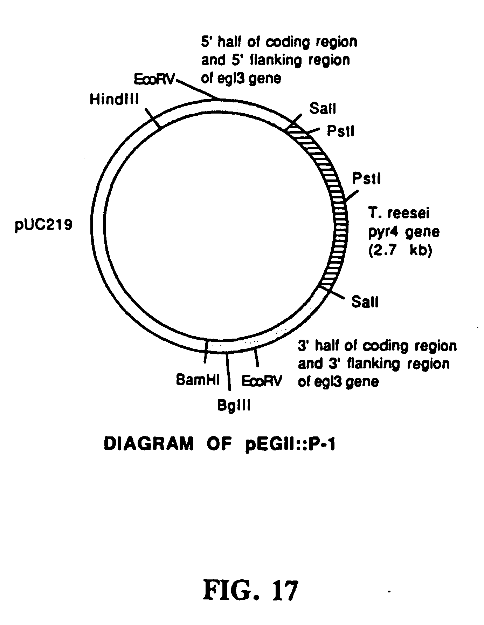

The egl3 gene, encoding EGII (previously referred to as EGIII by others), has been cloned from T. reesei and the DNA sequence published (Saloheimo et al., 1988, Gene 63:11-21). We have obtained the gene from strain RL-P37 as an approximately 4 kb PstI- XhoI fragment of genomic DNA inserted between the PstI and XhoI sites of pUC219. The latter vector, pUC219, is derived from pUC119 (described in Wilson et al., 1989, Gene 77:69-78) by expanding the multiple cloning site to include restriction sites for BgIII, ClaI and XhoI. Using methods known in the art the T. reesei pyr4 gene, present on a 2.7 kb SalI fragment of genomic DNA, was inserted into a SalI site within the EGII coding sequence to create plasmid pEGII::P-1 (FIG. 17). This resulted in disruption of the EGII coding sequence but without deletion of any sequences. The plasmid, pEGII::P-1 can be digested with HindII and BamHI to yield a linear fragment of DNA derived exclusively from T. reesei except for 5 bp on one end and 16 bp on the other end, both of which are derived from the multiple cloning site of pUC219.

Example 24

Transformation of T. reesei GC69 with pEGII::P-1 to creat a strain unable to produce EGII

T. reesei strain GC69 will be transformed with pEGII::P-1 which had been previously digested with HindIII and BamHI and stable transformants will be selected. Total DNA will be isolated from the transformants and Southern blot analysis used to identify those transformants in which the fragment of DNA containing the pyr4 and egl3 genes had integrated at the egl3 locus and consequently disrupted the EGII coding sequence. The transformants will be unable to produce EGII. It would also be possible to use pEGII::P-1 to transform a strain which was deleted for either or all of the cbh1, cbh2, or egl1 genes. In this way a strain could be constructed which would only produce certain cellulase components and no EGII component.

Example 25

Transformation of T. reesei with pEGII::P-1 to create a strain unable to produce CBHI. CBHII and EGII

A pyr4 deficient derivative of strain P37PΔΔCBH67 (from Example 11) was obtained by the method outlined in Example 1. This strain P37PΔΔ67P-1 was transformed with pEGII::P-1 which had been previously digested with HindIII and BamHI and stable transformants were selected. Total DNA was isolated from transformants and Southern blot analysis used to identify strains in which the fragment of DNA containing the pyr4 and eal3 genes had integrated at the egl3 locus and consequently disrupted the EGII coding sequence. The Southern blot illustrated in FIG. 18 was probed with an approximately 4 kb PstI fragment of T. reesei DNA containing the egl3 gene which had been cloned into the PstI site of pUC18 and subsequently re-isolated. When the DNA isolated from strain P37PΔΔ67P-1 was digested with PstI for Southern blot analysis the egl3 locus was subsequently visualized as a single 4 kb band on the autoradiograph (FIG. 18, lane E). However, for a transformant disrupted for the egl3 gene this band was lost and was replaced by two new bands as expected (FIG. 18, Lane F). If the DNA was digested with EcoRV or BglII the size of the band corresponding to the egl3 gene increased in size by approximately 2.7 kb (the size of the inserted pyr4 fragment) between the untransformed P37PΔΔ67P-1 strain (Lanes A and C) and the transformant disrupted for egl3 (FIG. 18, Lanes B and D). The transformant containing the disrupted egl3 gene illustrated in FIG. 18 (Lanes B, D and F) was named A22. The transformant identified in FIG. 18 is unable to produce CBHI, CBHII or EGII.

Example 26

Construction of pPΔEGI-1

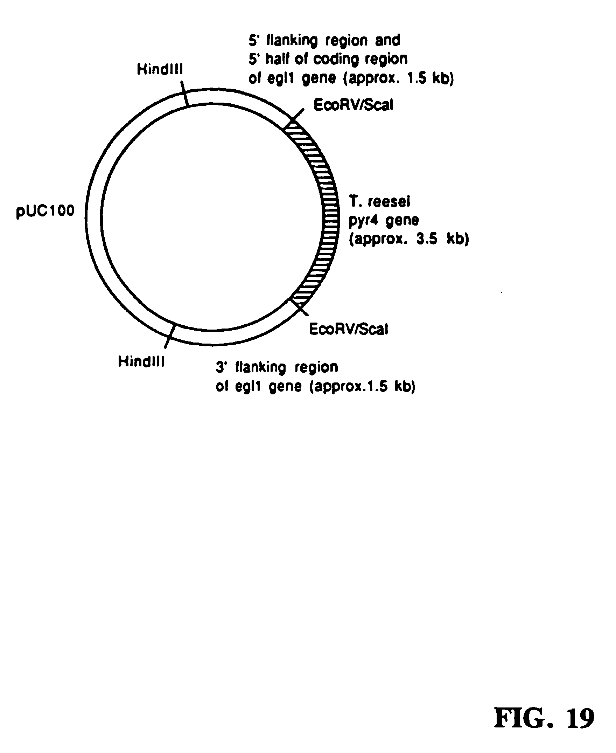

The egl1 gene of T. reesei strain RL-P37 was obtained, as described in Example 12, as a 4.2 kb HindIII fragment of genomic DNA. This fragment was inserted at the HindIII site of pUC100 (a derivative of pUC18:Yanisch-Perron et al., 1985, Gene 33:103-119, with an oligonucleotide inserted into the multiple cloning site adding restriction sites for BglII, ClaI and XboI). Using methodology known in the art an approximately 1 kb EcoRV fragment extending from a position close to the middle of the EGI coding sequence to a position beyond the 3' end of the coding sequence was removed and replaced by a 3.5 kb ScaI fragment of T. reesei DNA containing the pyr4 gene. The resulting plasmid was called pPAEGI-1 (see FIG. 19).
The plasmid, pPAEGI-1 can be digested with HindIII to release a DNA fragment comprising only T. reesei genomic DNA having a segment of the egl1 gene at either end and the pyr4 gene replacing part of the EGI coding sequence, in the center.
Transformation of a suitable T. reesei pyr4 deficient strain with the pPΔEGI-1 digested with HindIII will lead to integration of this DNA fragment at the egl1 locus in some proportion of the transformants. In this manner a strain unable to produce EGI will be obtained.

Example 27

Construction of pΔEGIpyr-3 and Transformation of a pyr4 deficient strain of T. reesei

The expectation that the EGI gene could be inactivated using the method outlined in Example 26 is strengthened by this experiment. In this case a plasmid, pΔEGIpyr-3, was constructed which was similar to pPΔEGI-1 except that the Aspergillus niger pyr4 gene replaced the T. reesei pyr4 gene as selectable marker. In this case the egl1 gene was again present as a 4.2 kb HindIII fragment inserted at the HindIII site of pUC100. The same internal 1 kb EcoRV fragment was removed as during the construction of pPΔEGI-1 (see Example 26) but in this case it was replaced by a 2.2 kb fragment containing the cloned A. niger pyrG gene (Wilson et al., 1988, Nucl. Acids Res. 16 p.2339). Transformation of a pyr4 deficient strain of T. reesei (strain GC69) with pΔEGIpyr-3, after it had been digested with HindIII to release the fragment containing the pyrG gene with flanking regions from the egl1 locus at either end, led to transformants in which the egl1 gene was disrupted. These transformants were recognized by Southern blot analysis of transformant DNA digested with HindIII and probed with radiolabelled pAEGIpyr-3. In the untransformed strain of T. reesei the egl1 gene was present on a 4.2 kb HindIII fragment of DNA and this pattern of hybridization is represented by Fig. 20, lane G. However, following deletion of the egl1 gene by integration of the desired fragment from pΔEGIpyr-3 this 4.2 kb fragment disappeared and was replaced by a fragment approximately 1.2 kb larger in size, FIG. 20, lane A. Also shown in FIG. 20, lane B is an example of a transformant in which integration of a single copy of pPΔEGIpyr-3 has occurred at a site in the genome other than the egl1 locus.

Example 28

Transformation of Treesei with pPΔEGI-1 to create a strain unable to produce CBHI, CBHII, EGI and EGII

A pyr4 deficient derivative of strain A22 (from Example 25) will be obtained by the method outlined in Example 1. This strain will be transformed with pPΔEGI-1 which had been previously digested with HindIII to release a DNA fragment comprising only T. reesei genomic DNA having a segment of the egl1 gene at either end with part of the EGI coding sequence replaced by the pyr4 gene.

Stable pyr4+ transformants will be selected and total DNA isolated from the transformants. The DNA will be probed with $^{32}$P labelled pPAEGI-1 after Southern blot analysis in order to identify transformants in which the fragment of DNA containing the pyr4 gene and egl1 sequences has integrated at the egl1 locus and consequently disrupted the EGI coding sequence. The transformants identified will be unable to produce CBHI, CBHII, EGI and EGII.

**Claims**

1.  Use of a detergent composition for washing a cotton-containing fabric, the composition comprising:

    (a) a cleaning effective amount of a surfactant or a mixture of surfactants; and.
    (b) from about 0.01 to about 5 weight percent of a fungal cellulase composition, wherein said cellulase composition comprises one or more endoglucanase components and less than about 5 weight percent of exo-cellobiohydrolase components based on the weight of protein in the cellulase composition;

    **characterized in that** washing the fabric with the composition provides improvements in softness, colour retention/restoration and feel, with reduced strength loss as compared to treatment with complete cellulase
    with the proviso that said fungal cellulase composition (b) is (i) other than a composition containing substantially pure EG III cellulase, being defined as having at least 40wt.% of EG III based on the total weight of cellulase proteins, and (ii) other than an acidic cellulase composition, ie having a pH optimum less than 7.0, containing an enriched amount of acidic EG type components relative to CBH type components.

2.  The use according to claim 1 wherein said detergent composition comprises from about 0.05 to about 2 weight percent of said cellulase composition.

3.  The use according to claim 1 wherein said cellulase composition is free of all exocellobiohydrolase components.

4.  The use according to claim 3 wherein said cellulase composition is derived from a genetically modified microorganism incapable of expressing any exo-cellobiohydrolase components and/or capable of overexpressing one or more endoglucanase components.

5.  The use according to claim 1 wherein said cellulase composition is derived from a genetically modified microorganism incapable of expressing any CBH type components.

6.  The use according to claim 5 wherein said cellulase composition does not contain any heterologous proteins.

**Patentansprüche**

1. Verwendung einer Waschmittel-Zusammensetzung zum Waschen von baumwollhältigem Gewebe, wobei die Zusammensetzung umfaßt:

    (a) eine Reinigung bewirkende Menge eines Tensids oder Tensidgemisches; und
    (b) etwa 0,01 bis etwa 5 Gew.-% einer Pilz-Cellulase-Zusammensetzung, bezogen auf das Gewicht der Waschmittelzusammensetzung, worin die Cellulase-Zusammensetzung eine oder mehrere Endoglucanase-Komponenten und weniger als etwa 5 Gew.-% an Exocellobiohydralase-Komponenten, bezogen auf das Gewicht des Proteins in der Cellulase-Zusammensetzung, umfaßt;

    **dadurch gekennzeichnet, daß** das Waschen des Gewebes mit der Zusammensetzung im Vergleich zu vollständiger Cellulase Verbesserungen bezüglich Weichheit, Farbechtheit/-wiederherstellung und Griff bereitstellt und für einen verringerten Festigkeitsverlust sorgt,
    mit der Maßgabe, dass die Pilz-Cellulase-Zusammensetzung (b) (i) keine Zusammensetzung ist, die im Wesentlichen reine EG-III-Cellulase enthält, was als Gehalt von zumindest 40 Gew.-% EG III, bezogen auf das Gesamtgewicht der Cellulaseproteine, definiert ist, und (ii) keine saure Cellulase-Zusammensetzung, d.h. mit einem pH-Optimum unter 7,0, ist, die relativ zu Komponenten vom CBH-Typ eine erhöhte Menge an Komponenten vom sauren EG-Typ enthält.

2. Verwendung nach Anspruch 1, worin die Waschmittel-Zusammensetzung etwa 0,05 bis etwa 2 Gew.-% der Cellulase-Zusammensetzung umfaßt.

3. Verwendung nach Anspruch 1, worin die Cellulase-Zusammensetzung frei von jeglichen Exocellobiohydrolase-Komponenten ist.

4. Verwendung nach Anspruch 3, worin die Cellulase-Zusammensetzung von einem genetisch modifizierten Mikroorganismus stammt, der nicht zum Exprimieren irgendwelcher Exocellobiohydrase-Komponenten fähig ist und/oder zum Überexprimieren einer oder mehrerer Endoglucanase-Komponenten fähig ist.

5. Verwendung nach Anspruch 1, worin die Cellulase-Zusammensetzung von einem genetisch modifizierten Mikroorganismus stammt, der nicht zum Exprimieren irgendwelcher CBH-Typ-Komponenten fähig ist.

6. Verwendung nach Anspruch 5, worin die Cellulase-Zusammensetzung keinerlei heterologe Proteine enthält.


**Revendications**

1. Utilisation d'une composition de détergent pour laver un tissu contenant du coton, la composition comprenant :

    (a) une quantité efficace pour le nettoyage d'un tensioactif ou d'un mélange de tensioactifs, et
    (b) d'environ 0,01 à environ 5% en poids d'une composition de cellulase fongique, dans laquelle ladite composition de cellulase comprend un ou plusieurs composants endoglucanase et moins d'environ 5% en poids de composants exo-cellobiohydrolase, sur base du poids de protéine dans la composition de cellulase,

    **caractérisée en ce que** le lavage du tissu avec la composition fournit des améliorations au niveau de la souplesse, de la rétention/restauration des couleurs et du toucher, avec une perte réduite de résistance par comparaison au traitement avec la cellulase complète,
    avec la condition que ladite composition de cellulase fongique (b) est (i) autre qu'une composition contenant la cellulase EG III sensiblement pure, définie comme ayant au moins 40% en poids d'EG III sur base du poids total des protéines cellulase, et (ii) autre qu'une composition de cellulase acide, à savoir ayant un pH optimal inférieur à 7,0, contenant une quantité enrichie en composants de type EG acide par rapport aux composants de type CBH.

2. Utilisation suivant la revendication 1, dans laquelle la composition de détergent se compose d'environ 0,05 à environ 2% en poids de la composition de cellulase.

3. Utilisation suivant la revendication 1, dans laquelle ladite composition de cellulase est exempte de tous les composants exo-cellobiohydrolase.

4. Utilisation suivant la revendication 3, dans laquelle ladite composition de cellulase dérive d'un microorganisme génétiquement modifié, incapable d'exprimer tout composant cellobiohydrolase et/ou capable de surexprimer un ou plusieurs composants endoglucanase.

5. Utilisation suivant la revendication 1, dans laquelle la composition de cellulase dérive d'un microorganisme génétiquement modifié, incapable d'exprimer un composant de type CBH quelconque.

6. Utilisation suivant la revendication 5, dans laquelle ladite composition de cellulase ne contient aucune protéine hétérologue.

FIG._1

FIG._2

FIG_3  FIG_4  FIG_5  FIG_7

EP 0 551 408 B2

FIG._6A

FIG._6B

FIG._8

FIG._9

EP 0 551 408 B2

FIG.-10

FIG._11

PANEL SCORE UNITS

pH

100 ppm

25 ppm

EP 0 551 408 B2

*FIG._12*

FIG._13

EP 0 551 408 B2

<u>cbh1</u>

```
AAACCCAATAGTCAACCGCGGACTGGCAT ATG TAT CGG
              G T A
AAACCCAATAGTGATCAGCGGACTGGCAT ATG TAT CGG
            BclI                 First 3 codons
```

<u>eg11</u>

```
TAGTCCTTCTTGTTGTCCCAAA ATG GCG CCC
              GGA
TAGTCCTTCTTGGGATCCCAAA ATG GCG CCC
            BamHI        First 3 codons
```

# FIG. 14

LINEAR FRAGMENT OF DNA OBTAINED FROM pCEPC1 BY EcoRI DIGESTION

18 bp from the pUC18 multiple cloning site
plus a 15 nt synthetic oligonucleotide

24 bp from the pUC19
multiple cloning site

EcoRI    BclI/BamHI    PstI    PstI    PstI    PstI    EcoRI

cbh1 promoter    egl1 coding sequence    T. reesei
(2.3 kb)    and terminator region    pyr4 (3.1 kb)
(1.9 kb)

(0.5 kb)    (1 kb)

cbh1 3' flanking
region

FIG. 15

40

FIG_16

DIAGRAM OF pEGII::P-1

FIG. 17

FIG_18

5' flanking region and
5' half of coding region
of egl1 gene (approx. 1.5 kb)

HindIII

EcoRV/ScaI

pUC100

T. reesei
pyr4 gene
(approx. 3.5 kb)

EcoRV/ScaI

HindIII

3' flanking region
of egl1 gene (approx.1.5 kb)

**FIG. 19**

FIG_20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 8909259 A
- US 4822516 A
- GB 2094826 A
- US 07422814 B
- US 07593919 B
- US 07625140 B
- US 07642669 B
- US 07642596 B
- US 4738682 A

### Non-patent literature cited in the description

- **Wood et al.** *Methods in Enzymology,* 1988, vol. 160 (25), 234
- **Coughlan et al.** Biochemistry and Genetics of Cellulose Degradation. Academic Press, 1988, 11
- **Wood et al.** Methods in Enzymology. Academic Press, 1988, vol. 160, 87
- **Wood.** *Biochem. Soc. Trans.,* 1985, vol. 13, 407-410
- **Wood et al.** *Biochemistry and Genetics of Cellulose Degradation,* 1988, 31-52
- **Wood et al.** *Carbohydrate Research,* 1989, vol. 190, 279-297
- **Schulein.** *Methods in Enzymology,* 1988, vol. 160, 234-242
- **Miller et al.** Direct and Indirect Gene Replacement in Aspergillus nidulans. *Molecular and Cellular Biology,* 1985, 1714-1721
- **Coughlan et al.** Biochemistry and Genetics of Cellulose Degradation. Academic Press, 1988, 11-30
- **Sheir-Neiss, G ; Montenecourt, B.S.** *Appl. Microbiol. Biotechnol.,* 1984, vol. 20, 46-53
- **Chen et al.** *Biotechnology,* vol. 5, 274-278
- **Smith et al.** *Curr. Genet,* 1991, vol. 19, 27-33
- **Penttila et al.** *Gene,* vol. 45, 253-263
- **van Arsdell et al.** *Bio/Technology,* 1987, vol. 5, 60-64
- **Sheir-Neiss ; Montenecourt.** *Appl. Microbiol. Biotechnol.,* 1984, vol. 20, 46-53
- **Saloheimo et al.** *Gene,* 1988, vol. 63, 11-21
- **Wilson et al.** *Gene,* vol. 77, 69-78
- **Yanisch-Perron et al.** *Gene,* vol. 33, 103-119